(19) **Europäisches Patentamt / European Patent Office / Office européen des brevets**

(11) **EP 4 487 856 A1**

(12) # EUROPEAN PATENT APPLICATION

(43) Date of publication:
**08.01.2025 Bulletin 2025/02**

(21) Application number: **23184673.4**

(22) Date of filing: **11.07.2023**

(51) International Patent Classification (IPC):
*A61K 35/00* (2006.01)     *A61P 1/00* (2006.01)

(52) Cooperative Patent Classification (CPC):
**A61K 35/00; A61P 1/00;** A61K 2035/115

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **07.07.2023 PT 2023118810**

(71) Applicant: **Fundação Calouste Gulbenkian**
**1067-001 Lisbon (PT)**

(72) Inventors:
• **DE BIVAR XAVIER, Karina**
**1000-129 Lisbon (PT)**
• **RODRIGUES CABRAL, Vitor Hugo**
**2735-655 Lisbon (PT)**
• **ALMEIDA DE OLIVEIRA, Ana Rita**
**1067-001 Lisbon (PT)**

(74) Representative: **Hutter, Anton**
**Venner Shipley LLP**
**5 Stirling House**
**Stirling Road**
**The Surrey Research Park**
**Guildford GU2 7RF (GB)**

(54) **BACTERIAL COMPOSITION**

(57)     The invention relates to probiotic compositions comprising viable bacteria of *Klebsiella* strain ARO112, optionally for use as a therapeutic medicament. Compositions of the invention are provided for use in preventing, or treating dysbiosis, intestinal inflammation, infection with intestinal pathogens, or intestinal inflammatory diseases such Crohn's disease, or ulcerative colitis.

EP 4 487 856 A1

**Description**

Field

The present invention relates to a probiotic composition comprising *Klebsiella* strain ARO112, and a probiotic composition for use as a medicament, particularly in the prevention or treatment of dysbiosis, intestinal inflammation or bacterial infection.

*Background*

Inflammatory Bowel Diseases (IBD) are a group of multi-factorial pathologies with an etiology involving genetic predisposition, and/or environmental influences. The limited treatment options available focus on the common symptoms that greatly diminish patients' quality of life. Three symptoms emerge as the core of a disease cycle that afflict IBD patients, increased and persistent intestinal inflammation, gut microbiota imbalances, and susceptibility to recurrent intestinal infection.

Intestinal inflammatory flares are frequent in IBD, for which patients take antibiotics or anti-inflammatory medication. These drugs however, can have a detrimental effect on the intestinal microbiota, and may exacerbate a proinflammatory intestinal environment (Santana, 2022). An additional effect of an imbalanced microbiota is the consequent loss of natural protection that a balanced microbiota provides and subsequent increase in susceptibility to infection, often by *Enterobacteriaceae* bacteria, like the adherent-invasive *Escherichia coli* (AIEC), which can also induce inflammatory bouts (Small, 2013). The gold-standard treatment against bacterial infections is still antibiotic therapy, which leads to further intestinal microbiota imbalances with consequent increase in susceptibility to infection.

This intricate and self-sustained disease cycle exponentiates the chronicity of IBD, leaving patients constantly in a disease-treatment-disease state, in need of novel and efficient approaches that can complement current treatments disrupting this burdening cycle. Probiotics have long been suggested as complementary or alternative approaches to current treatments for intestinal diseases, but few cases have been shown to be efficient for complex pathologies, like IBD. Probiotic bacteria have been suggested for IBD treatment (Carmen, 2011), however, current probiotics are not known to target the diverse symptoms described above to be critical for IBD. Furthermore, therapeutic probiotics have encountered lack of efficacy when tested in patients, rather than in laboratory conditions, which can be explained by some frequent caveats: 1) probiotic strains are foreign to the target microbiota, therefore unable to colonize long enough to actuate effects; 2) probiotic bacteria persistently colonize imbalanced microbiota communities, not allowing for natural microbiota recovery of diversity to occur (Suez, 2018); 3) probiotic bacterial strains can contribute to the increase of antibiotic resistance transfer (Montassier, 2021). Probiotics have been shown to delay microbiota recovery after antibiotic treatment, lingering in the microbiota for long periods of time (Suez, 2018). Furthermore, AIEC infection has been shown to hinder microbiota recovery in IBD, even upon a strong therapy, like fecal microbiota transplant (Zhilu 2021).

*Escherichia coli* Nissle 1917 (EcN) has long been used as probiotic strain, and has been touted as a potential therapy for IBD (Schultz, 2008). The common probiotic strain EcN has shown partial effect on AIEC growth in *in vitro* assays (Huebner 2011). None of the aforementioned probiotic therapies were tested in an inflammatory context, which is particularly susceptible to dysbiosis, nor have been studied in the presence of AIEC, which, as mentioned above, drives lingering dysbiosis and hinders recovery. One recent approach found promising results with a GMO EcN strain in an inflammatory context, however genetically modified organisms face steep regulatory hurdles (Zhou 2022).

In previous work, the inventors identified a commensal intestinal bacterium, *Klebsiella* sp. ARO112, that provided limited colonization resistance against colonization with a *E. coli* K-12 MG1655 strain in a streptomycin-induced intestinal dysbiosis murine model (Oliveira, 2020). However, the effect on pathogen numbers was transient, and as with other probiotics, it was unclear whether such displacement could be achieved in an inflammatory environment resembling an IBD patient, or whether the intervention had a positive or negative impact on microbial diversity, and local inflammation. Furthermore, *Klebsiella* sp. ARO112 is a member of the *Klebsiella* genus comprising many opportunistic pathogens such as *K. pneumoniae* and *K. oxytoca,* and the safety regarding pathogenesis and virulence of the *Klebsiella* sp. ARO112 is unknown. For *Enterobacteriaceae* in general, one of the most challenging and problematic characteristics is the easy acquisition of antibiotic resistances from other members of the community, namely by horizontal gene transfer events, like plasmid conjugation. This is evident even in non-pneumoniae *Klebsiella* species (Moradigaravand 2017; Luo 2022; Hubbard 2020), making them unsuited to probiotic or therapeutic use.

Based on the above-mentioned state of the art, the objective of the present invention is to provide means and methods to prevent or treat dysbiosis, and/or intestinal inflammation, or diseases associated with the above such as IBD, and pathogenic bacterial infections. This objective is attained by the subject-matter of the independent claims of the present specification, with further advantageous embodiments described in the dependent claims, examples, figures and general description of this specification.

Summary of the Invention

This study demonstrates how a protective bacterial species, *Klebsiella* sp. ARO112, competes with other *Enterobacteriaceae* and inhibits intestinal inflammation. It does this with little production of inflammatory siderophores, while remaining tolerant of host lipocalin-2 production, therefore being potentially suited to compete with invading bacteria in inflammatory environments without further enhancing inflammation. Furthermore, ARO112 also shows a strong attenuation in both antibiotic resistance acquisition and retention, usually associated with Enterobacteriaceae clinical isolates, like Kp1012, but also in EcN, highlighting how this commensal strain is potentially safer for use as a probiotic, or therapeutic species.

Studies in animal models demonstrate ARO112 is a successful probiotic therapy for IBD, targeting several microbiota-based disease hallmarks. It displaces invading pathobionts, including clinically relevant, AIEC; promotes recovery of the diverse microbiota, in particular reducing *Proteobacteria*; and increases the proportion of butyrate-producers, attenuating the possibility of pathobiont blooms while inhibiting inflammatory responses, respectively.

The ensemble of these results, namely the displacement of infectious agents such as AIEC, the non-inducing of inflammation, and the recovery of microbiota, all obtained upon treatment with ARO112, validate a three-pronged therapy targeting the three hallmarks of IBD.

A first aspect of the invention relates to a probiotic composition comprising viable bacteria *Klebsiella* sp. ARO112 as the active ingredient, for use as a probiotic for oral or enteral administration. The probiotic according to the invention is particularly suited to administration to subjects having recently received a course of antibiotic therapy, or who suffer from intestinal inflammatory disease such as ulcerative colitis, or Crohn's disease.

A second aspect of the invention relates to a probiotic composition comprising viable bacteria *Klebsiella* sp. ARO112 as the active ingredient, formulated for oral or enteral administration, for use in treating, or preventing a recurrence of inflammatory conditions, including intestinal dysbiosis, intestinal inflammatory diseases, infection with intestinal bacterial pathogens such as *Enterobacteriaceae,* and/or inflammatory bowel disease flare-ups.

## *Terms and definitions*

For purposes of interpreting this specification, the following definitions will apply and whenever appropriate, terms used in the singular will also include the plural and vice versa. In the event that any definition set forth below conflicts with any document incorporated herein by reference, the definition set forth shall control.

The terms "comprising", "having", "containing", and "including", and other similar forms, and grammatical equivalents thereof, as used herein, are intended to be equivalent in meaning and to be open-ended in that an item or items following any one of these words is not meant to be an exhaustive listing of such item or items, or meant to be limited to only the listed item or items. For example, an article "comprising" components A, B, and C can consist of (i.e., contain only) components A, B, and C, or can contain not only components A, B, and C but also one or more other components. As such, it is intended and understood that "comprises" and similar forms thereof, and grammatical equivalents thereof, include disclosure of embodiments of "consisting essentially of" or "consisting of."

Where a range of values is provided, it is understood that each intervening value, to the tenth of the unit of the lower limit, unless the context clearly dictates otherwise, between the upper and lower limit of that range and any other stated or intervening value in that stated range, is encompassed within the disclosure, subject to any specifically excluded limit in the stated range. Where the stated range includes one or both of the limits, ranges excluding either or both of those included limits are also included in the disclosure.

Reference to "about" a value or parameter herein includes (and describes) variations that are directed to that value or parameter per se. For example, description referring to "about X" includes description of "X."

As used herein, including in the appended claims, the singular forms "a", "or" and "the" include plural referents unless the context clearly dictates otherwise.

"And/or" where used herein is to be taken as specific recitation of each of the two specified features or components with or without the other. Thus, the term "and/or" as used in a phrase such as "A and/or B" herein is intended to include "A and B," "A or B," "A" (alone), and "B" (alone). Likewise, the term "and/or" as used in a phrase such as "A, B, and/or C" is intended to encompass each of the following aspects: A, B, and C; A, B, or C; A or C; A or B; B or C; A and C; A and B; B and C; A (alone); B (alone); and C (alone).

Unless defined otherwise, all technical and scientific terms used herein have the same meaning as commonly understood by one of ordinary skill in the art (e.g., in cell culture, molecular genetics, nucleic acid chemistry, hybridization techniques and biochemistry, organic synthesis). Standard techniques are used for molecular, genetic, and biochemical methods (see generally, Sambrook et al., Molecular Cloning: A Laboratory Manual, 4th ed. (2012) Cold Spring Harbour Laboratory Press, Cold Spring Harbour, N.Y. and Ausubel et al., Short Protocols in Molecular Biology (2002) 5th Ed, John Wiley & Sons, Inc.) and chemical methods.

The term *probiotic* in the context of the present specification relates to pharmaceutical composition comprising live bacteria, or lyophilized bacteria which stimulates the growth of microorganisms with beneficial properties in the intestine of the recipient.

The terms *LF82, E. coli LF82* in the context of the present specification relates to *E. coli* pathobiont AIEC strain commonly associated with IBD patients. This strain can be identified by the Taxonomy ID 591946 in the NCBI taxonomy browser, and the genome is available in several databases.

The term *inflammatory intestinal disease* in the context of the present specification relates to inflammatory bowel diseases such as Crohn's disease (CD), or ulcerative colitis (UC). Further conditions associated with intestinal inflammation include for example, obesity, malnutrition, and aging.

The term *viable* in the context of the present specification relates to living bacteria. This can be confirmed by means known in the art such as plating of progressive dilutions on agar plates, or alternatively, can be assayed using cell counting machines and live-dead discriminating dye. The term encompasses lyophilised, or freeze-dried bacteria, which can resuscitate and grow when hydrated.

As used herein, the term *pharmaceutical composition* refers to a composition of viable bacteria according to the invention, together with at least one pharmaceutically acceptable carrier. In certain embodiments, the pharmaceutical composition according to the invention is provided in a form suitable for topical, parenteral or injectable administration.

As used herein, the term *pharmaceutically acceptable carrier* includes any solvents, dispersion media, coatings, surfactants, antioxidants, preservatives (for example, antibacterial agents, antifungal agents), isotonic agents, absorption delaying agents, salts, preservatives, drugs, drug stabilizers, binders, excipients, disintegration agents, lubricants, sweetening agents, flavoring agents, dyes, and the like and combinations thereof, as would be known to those skilled in the art (see, for example, Remington: the Science and Practice of Pharmacy, ISBN 0857110624).

As used herein, the term *treating* or *treatment* of any disease or disorder (e.g. an infection with AIEC) refers in one embodiment, to ameliorating the disease or disorder (e.g. slowing or arresting or reducing the development of the disease or at least one of the clinical symptoms thereof). In another embodiment "treating" or "treatment" refers to alleviating or ameliorating at least one physical parameter including those which may not be discernible by the patient. In yet another embodiment, "treating" or "treatment" refers to modulating the disease or disorder, either physically, (e.g., stabilization of a discernible symptom), physiologically, (e.g., stabilization of a physical parameter), or both. Methods for assessing treatment and/or prevention of disease are generally known in the art, unless specifically described herein.

*Sequences*

Sequences similar or homologous (e.g., at least about 70% sequence identity) to the sequences disclosed herein are also part of the invention. In some embodiments, the sequence identity at the amino acid level can be about 80%, 85%, 90%, 91%, 92%, 93%, 94%, 95%, 96%, 97%, 98%, 99% or higher. At the nucleic acid level, the sequence identity can be about 70%, 75%, 80%, 85%, 90%, 91%, 92%, 93%, 94%, 95%, 96%, 97%, 98%, 99% or higher. Alternatively, substantial identity exists when the nucleic acid segments will hybridize under selective hybridization conditions (e.g., very high stringency hybridization conditions), to the complement of the strand. The nucleic acids may be present in whole cells, in a cell lysate, or in a partially purified or substantially pure form.

In the context of the present specification, the terms *sequence identity* and *percentage of sequence identity* refer to a single quantitative parameter representing the result of a sequence comparison determined by comparing two aligned sequences position by position. Methods for alignment of sequences for comparison are well-known in the art. Alignment of sequences for comparison may be conducted by the local homology algorithm of Smith and Waterman, Adv. Appl. Math. 2:482 (1981), by the global alignment algorithm of Needleman and Wunsch, J. Mol. Biol. 48:443 (1970), by the search for similarity method of Pearson and Lipman, Proc. Nat. Acad. Sci. 85:2444 (1988) or by computerized implementations of these algorithms, including, but not limited to: CLUSTAL, GAP, BESTFIT, BLAST, FASTA and TFASTA. Software for performing BLAST analyses is publicly available, e.g., through the National Center for Biotechnology-Information (http://blast.ncbi.nlm.nih.gov/).

One example for comparison of amino acid sequences is the BLASTP algorithm that uses the default settings: Expect threshold: 10; Word size: 3; Max matches in a query range: 0; Matrix: BLOSUM62; Gap Costs: Existence 11, Extension 1; Compositional adjustments: Conditional compositional score matrix adjustment. One such example for comparison of nucleic acid sequences is the BLASTN algorithm that uses the default settings: Expect threshold: 10; Word size: 28; Max matches in a query range: 0; Match/Mismatch Scores: 1.-2; Gap costs: Linear. Unless stated otherwise, sequence identity values provided herein refer to the value obtained using the BLAST suite of programs (Altschul et al., J. Mol. Biol. 215:403-410 (1990)) using the above identified default parameters for protein and nucleic acid comparison, respectively.

Reference to identical sequences without specification of a percentage value implies 100% identical sequences (i.e. the same sequence).

The term *having substantially the same biological activity* in the context of the present invention relates to either one or both main functions of a ARO112 composition, i.e. displacement of the AIEC strain in the inflammatory Nod2-/- mouse model of IBD. In brief, an ARO112 composition having substantially the same biology activity as the ARO112 strain characterised by the genome deposited in the Sequence Read Archive (SRA) NCBI database under BioProject ID. PRJNA590204, displaces the pathogen AIEC strain in under 12 days, using the methodology described in the section *Animal Experiments* section of the *Materials and Methods* provided below.

## Detailed Description of the Invention

The inventors have demonstrated an unexpected and unique capacity of the *Klebsiella sp.* strain ARO112 to enhance intestinal microbial diversity and dampen intestinal inflammation. *Klebsiella* sp. strain ARO112 is a non-spore forming facultative anaerobe. The inventors demonstrate herein that this bacterial species is surprisingly characterized by a high safety profile, as it is generally resistant to acquisition of antibiotic resistance genes, promotes growth of anti-inflammatory butyrate-producing bacteria, and is gradually eliminated from the intestine following oral administration.

### Probiotic ARO 112 compositions

One aspect of the invention relates to a probiotic composition comprising essentially (in other words, comprising as the only active bacterial ingredient) viable *Klebsiella sp.* strain ARO112. Probiotics are bacterial cultures consumed orally to enhance intestinal health. In some embodiments, probiotics compositions according to the invention are formulations of viable *Klebsiella sp.* strain ARO112 together with buffers or excipients. In certain embodiments probiotic compositions according to the invention are in the form of conveniently packaged probiotic foodstuffs, such as dairy products, or food supplements, such as vitamins or calcium, or fibre, supplement with viable colony-forming units of *Klebsiella sp.* strain ARO112. In some embodiments, a probiotic according to the current invention takes the form of packaged food items manufactured to provide a dose of active, viable *Klebsiella sp.* strain ARO112 bacteria in single serving portion, for example, 100 million total viable cells in a serving portion.

Another aspect of the invention relates to probiotic compositions comprising as the only active bacterial ingredient viable *Klebsiella sp.* strain ARO112, for use as a medicament.

In certain embodiments of the probiotic composition, it comprises a *Klebsiella* bacterial strain characterized by a genome more than (>) 97% identical to that of the *Klebsiella* strain ARO112 genome deposited in the Sequence Read Archive (SRA) NCBI database under BioProject ID. PRJNA590204.

In some embodiments, the probiotic composition comprises a *Klebsiella* ARO112 strain with a genome >97% >98%, >99%, >99.5%, or particularly >99.9% similar to that described above, and has the same substantially the same biological activity, i.e. the capacity to prevent long-term engraftment of pathogenic *Enterobacteriaceae.*

In some embodiments of the probiotic ARO112 composition for use as a medicament, the composition comprises a *Klebsiella sp.* ARO112 strain derived from the strain characterised by the genome deposited in the Sequence Read Archive (SRA) NCBI database under BioProject ID. PRJNA590204.

In particular embodiments of the probiotic ARO112 composition according the invention, it is provided in a form suited to oral administration, such as a capsule comprising a bacterial formulation, or lyophilized bacteria. It is understood that in addition to live *Klebsiella* strain ARO112 bacteria, the probiotic composition may also contain a certain percentage of dead bacteria,

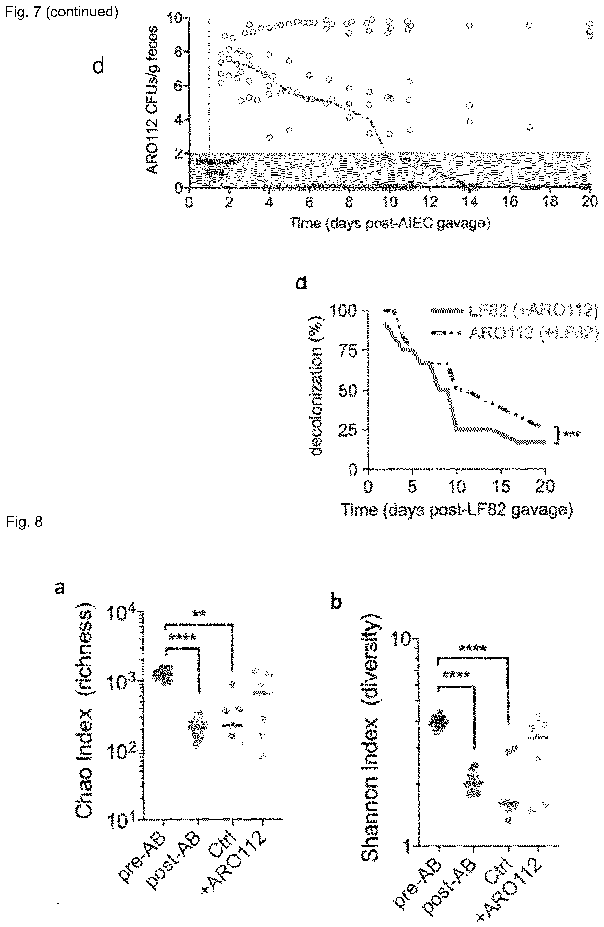

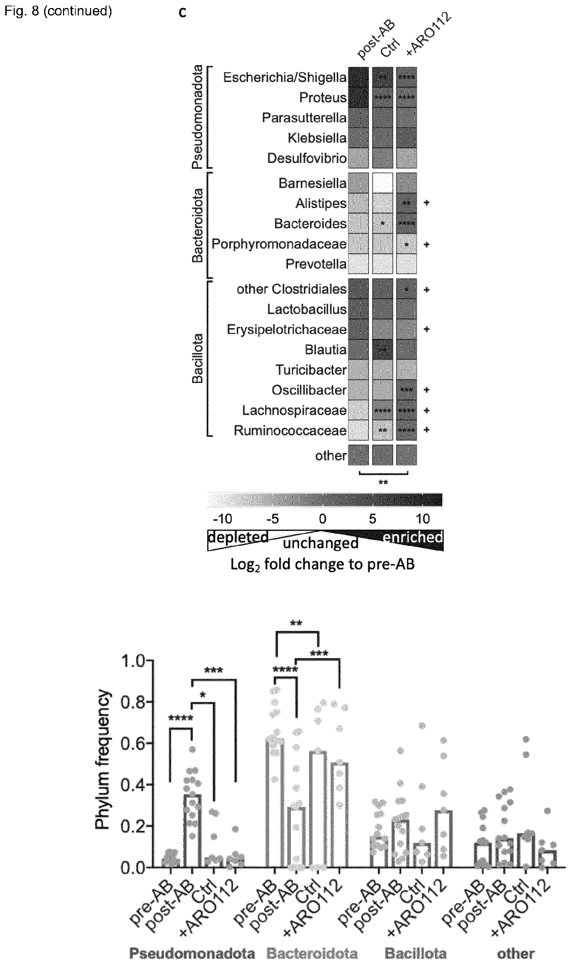

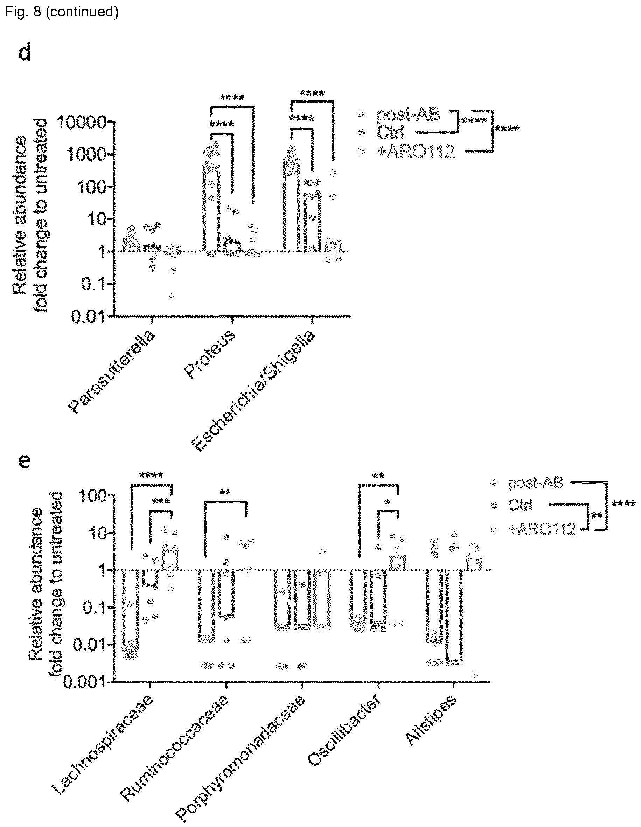

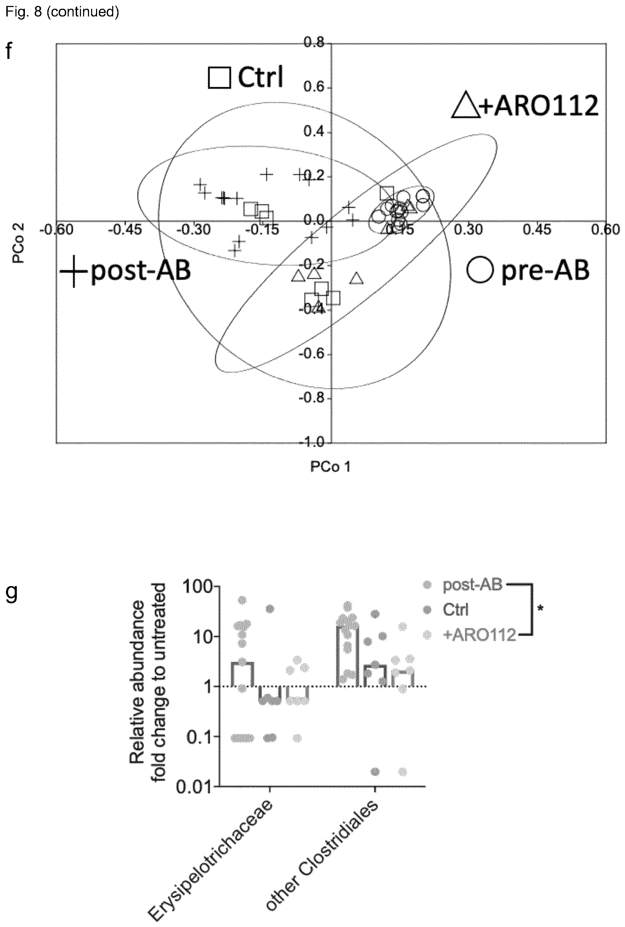

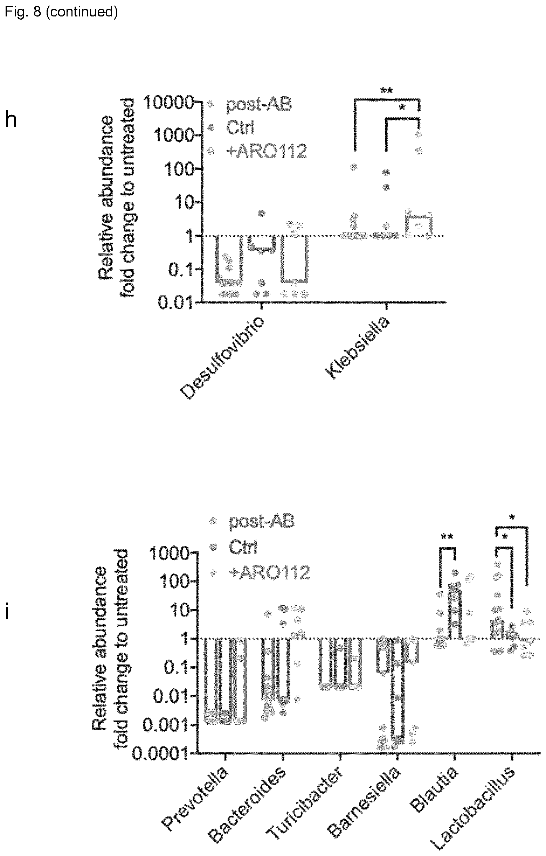

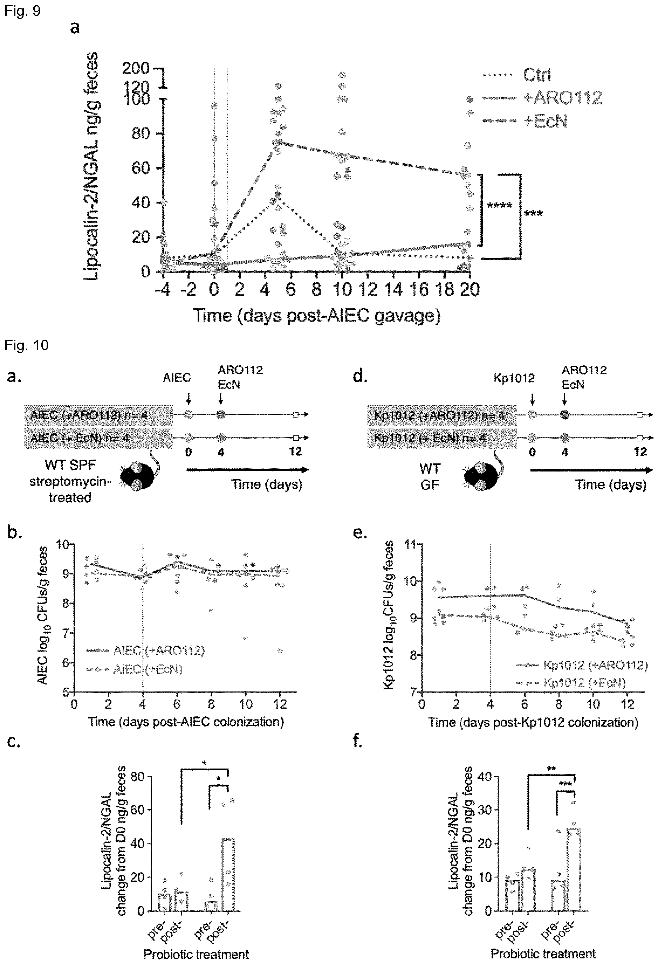

In some embodiments of the probiotic ARO112 composition for use as a medicament, the composition is provided to promote bacterial diversity in a mammalian subject, particularly a human subject. Figures 8 and 9 in the Examples demonstrate that a composition according to the invention can enhance microbial diversity in general, particularly members of beneficial butyrate-producing classes of bacteria.

In certain embodiments, the bacterial composition for treatment or prevention of recurrence of cancer is composed of isolated live bacteria. "Isolated" in this context refers to bacteria which are not part of a faecal transplant but have been produced by separating, or isolating bacteria from a natural source (and optionally, culturing them). In particular embodiments, the probiotic bacterial composition is free of faecal matter. In other words, the composition contains isolated bacterial strains that have been produced in an industrial setting, rather than being isolated or cultured from human faecal samples.

### Probiotic ARO 112 compositions for preventing or treating intestinal dysbiosis and/or inflammation

In some embodiments of the probiotic ARO112 composition for use as a medicament according to the invention, the ARO112 composition is provided for use in the prevention, or treatment of intestinal dysbiosis in a mammalian subject, particularly a human subject.

Intestinal dysbiosis refers to a range of conditions comprising, but not being limited to, abdominal pain, diarrhoea, discomfort after eating, and malnutrition, are associated with intestinal dysbiosis (also referred to as dysbacteriosis). Dysbiosis is in turn caused by a disruption to the microbiome resulting in an imbalance in the intestinal microbiota, changes in their functional composition and metabolic activities, or a shift in their local distribution. Prevention of dysbiosis may be particularly desirable in a subject having recently received a course of antibiotics. Diagnosis of dysbiosis, and subsequent treatment may involve treatment of the symptoms described above, or may be based on an analysis of the faecal microbiome providing a diagnosis of dysbiosis.

In some embodiments of the probiotic ARO112 composition for use as a medicament according to the invention, the ARO112 composition is provided for use in preventing, or treating intestinal inflammation in a mammalian subject, particularly a human subject. Intestinal inflammation encompasses a range of conditions, most notably inflammatory bowel disease such as Crohn's disease, or ulcerative colitis. Further conditions associated with intestinal inflammation include for example, obesity, malnutrition, and aging, similarly associated with a range of symptoms comprising, but not being limited to, abdominal pain, diarrhoea, discomfort after eating, and malnutrition, bloating, and intestinal tissue damage and bleeding.

*Probiotic ARO 112 compositions for use preventing or treating intestinal bacterial infection*

In some embodiments of the probiotic ARO112 composition for use as according the invention, it is provided for prevent an intestinal infection with pathogenic bacteria. This can be particularly relevant in a certain high-risk patients, for example a patient having an intestinal inflammation disorder such as IBD, or a recent medical history of intestinal bacterial infection (for example, within the previous year, or particularly the previous 2 months), or post-antibiotic dysbiosis and associated discomforting symptoms.

In some embodiments, a probiotic ARO112 composition may be administered prior to a hospital stay, to deter nosocomial infections by enriching intestinal microbial diversity. In some embodiments, the probiotic ARO112 composition is provided for use subsequent to a course of antibiotics, particularly oral antibiotics, to prevent, or more quickly resolve pathogenic infections arising as the microbiota recovers. In some embodiments of the probiotic composition according to the invention for use as a medicament, it is provided to a patient who has been diagnosed with a pathogenic bacterial infection. In particular embodiments, the infectious bacteria is a member of the *Enterobacteriaceae* family

In particular embodiments, the pathogenic bacterial species is an infectious agent commonly associated with inflammatory conditions characterised by persistent intestinal bacterial infections. In some embodiments, the infection is caused by a is characterised as resistant to antibiotic treatments, for example an antibiotic-resistant adherent invasive *Escherichia coli* (*Ecoli*), or AIEC, a multi-drug-resistant species of *E.coli, Klebsiella pneumoniae.*

In certain embodiments, the pathogen is a bacterial species that commonly infects patients following antibiotics, such *as Vibrio cholerae* or *Clostridioides difficile.*

In certain embodiments of the probiotic composition for use as a medicament according to the invention, it is provided for use in preventing, or treating an intestinal infection with an antibiotic-resistant bacterial species. In particular embodiments, the ARO112 composition is provided for use in preventing, or treating an infection with a multi-drug resistant bacterial species.

In particular embodiments of the ARO112 composition for use according the aspects of the invention listed above, it is provided for use in treating, or preventing an infection caused by a common nosocomial pathogenic bacterial species. Preventative treatment may be particularly useful to prevent a recurrent infections, provided to a subject within the weeks or months leading up to a scheduled hospital stay.

In some embodiments, the probiotic composition according to the invention is provided for use in preventing or treating an infection caused by a pathogenic bacterial species of the genus *Escherichia, Citrobacter,* or *Salmonella.*

In alternative embodiments, the probiotic ARO112 composition is provided for use in preventing, or treating a subject with an infection caused by *Clostridioides difficile.*

In certain embodiments, the probiotic ARO112 composition is provided for use in preventing, or treating intestinal infection caused by a vancomycin-resistant *Enterococcus* species.

In certain embodiments of the probiotic ARO112 composition, it is provided to prevent, or treat an intestinal infection by a member of the clinically-relevant ESKAPEE multi-drug resistant species selected from *Enterococcus faecium, Staphylococcus aureus, Klebsiella pneumoniae, Acinetobacter baumannii, Pseudomonas aeruginosa, Enterobacter species,* and *E. coli.*

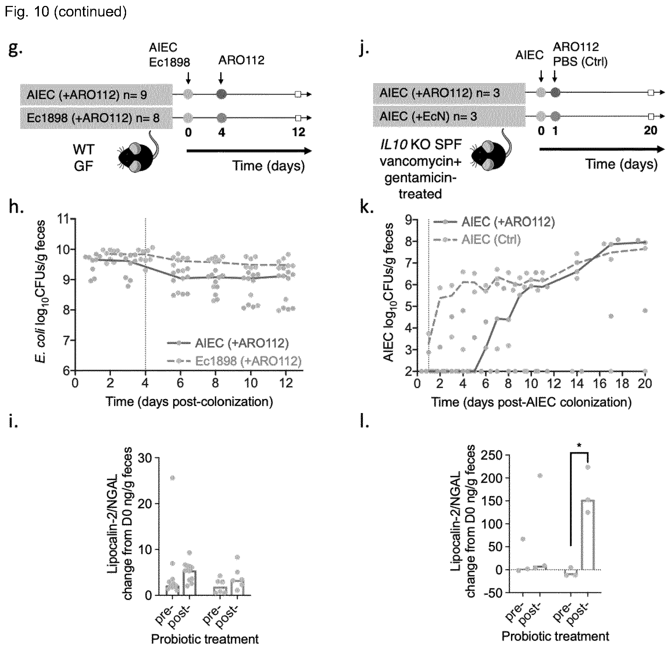

In particular embodiments, the probiotic ARO112 composition is provided for use in preventing, or treating an intestinal infection by a strain of adherent-invasive *E. coli* (AIEC). Such species which can attach to the mucosal layer of the gut (ileum and colon) particularly in IBD patients (CD and UC) and cause mucosal damage and inflammation, leading to the typical inflammatory flares. These infections are common in IBD patients, particularly in CD (Rolhion 2007), as modelled by the Nod2-/- mice for which ARO112 provided beneficial anti-inflammatory effects in the data presented herein. In particular embodiments, the probiotic composition is provided to treat an infection with the AIEC strain LF82, a prototype strain of the pathotype AIEC, which is completely displaced by a single dose of a composition according to the invention (Fig. 9, 10). This strain has been clinically isolated worldwide, and is the most common pathogenic strain associated with IBD patients (Palmela 2018).

In certain embodiments, the ARO112 composition is provided for use in preventing, or treating an intestinal infection caused by a pathogenic bacterial species classified as an enterohemorrhagic *Escherichia coli* (EHEC), enteroaggregative *E. coli (EAEC),* enteropathogenic *E. coli (EPEC),* or uropathogenic *E. coli* (*UPEC*).

*Probiotic ARO 112 compositions for use treating antibiotic-related pathology*

In certain embodiments, the probiotic ARO112 composition is provided for use for use in a mammalian subject, particularly a human subject subsequent to a therapeutic course of antibiotics. In particular embodiments, the subject has finished a course of antibiotics in the previous month. In more particular embodiments, the subject has been administered the final dose of antibiotics in the 48 hours prior to administration of the ARO112 composition. The inventors have successfully tested a single dose of probiotic 48h after last antibiotic dose to prevent subsequent establishment of an *Enterobacteriaceae,* or *V. cholerae* infection. In alternative embodiments, the ARO112 composition is provided for use to therapeutically treat a patient with recurrent dysbiotic symptoms persisting long after antibiotic treatment. In particular embodiments, the patient has diagnosis of an inflammatory intestinal disease, such as IBD, and has received antibiotics to treat a flare-up symptoms.

In certain embodiments of the ARO112 composition for use as a probiotic, or medicament, it is provided for use in a patient subsequent to a therapeutic course of antibiotics comprising vancomycin, gentamicin, metronidazole, ciprofloxacin, and/or rifaximin. In certain embodiments, the composition is provided for use to counter dysbiosis following treatment of a patient with a combination of vancomycin and gentamicin, or metronidazole, and ciprofloxacin, commonly prescribed to IBD patients. Vancomycin and gentamicin have been successfully tested in a cohort of infants with early onset IBD, therefore, it is a viable and proved therapeutic approach for IBD (Lev-Tzion 2017). Metronidazole and ciprofloxacin (alone or in combination), and rifaximin have been used in IBD, and further clinically effective combinations are known in the art (Xiong 2015). An ARO112 composition is susceptible to antibiotics, and it is understood that administration prior to, or subsequent to doses of antibiotics is most suitable.

*Probiotic ARO 112 compositions for use in treatment of inflammatory intestinal disease*

In certain embodiments of the probiotic composition comprising strain ARO112 for use as a medicament, it is provided for use in a mammalian subject, particularly a human subject, or patient diagnosed with an intestinal inflammatory disease. This encompasses a range of conditions most notably inflammatory bowel diseases such as Crohn's disease, or ulcerative colitis. Further conditions associated with intestinal inflammation include for example, obesity, malnutrition, and aging.

In some embodiments, the probiotic ARO112 composition is provided for use in a patient diagnosed with IBD/inflammation disease as well as an additional clinical condition described herein, such as a pathogenic bacterial infection, or post-antibiotics dysbiosis specifically to the context of a patient with IBD. The efficacy of this strain in reducing inflammation in IBD patients is modelled by Nod2-/- mice in the Examples, and is surprising in view of findings that probiotics are understood to exacerbate disease specifically in IBD patients

In certain embodiments of the probiotic composition, it is provided for use in treating a patient with inflammatory bowel disease, i.e reducing their symptoms, reducing markers of dysbiosis or intestinal pathology, or reducing the frequency of associated intestinal infections. In particular embodiments, the disease is Crohn's disease. In further particular embodiments, the disease is ulcerative colitis.

*Medical treatment*

Similarly, within the scope of the present invention is a method for treating dysbiosis, intestinal inflammation, or an inflammatory intestinal disease in a patient in need thereof, comprising administering to the patient a probiotic composition comprising viable *Klebsiella sp.* strain ARO112 according to the above description.

*Pharmaceutical Compositions, Administration/Dosage Forms and Salts*

According to one aspect of the probiotic composition according to the invention, it is provided as a pharmaceutical composition, pharmaceutical administration form, or pharmaceutical dosage form. Said pharmaceutical composition, pharmaceutical administration form, or pharmaceutical dosage form compriseslive *Klebsiella* strain ARO112 bacteria of the present invention and at least one pharmaceutically acceptable carrier, diluent or excipient.

In certain embodiments of the invention, the live *Klebsiella* strain ARO112 bacteria of the present invention is typically formulated into pharmaceutical dosage forms to provide an easily controllable dosage of the drug and to give the patient an elegant and easily handleable product.

The invention further encompasses a pharmaceutical composition comprising the live *Klebsiella* strain ARO112 bacteria of the present invention, and a pharmaceutically acceptable carrier. In further embodiments, the composition comprises at least two pharmaceutically acceptable carriers, such as those described herein.

Certain embodiments of the invention relate to a dosage form for enteral administration, such as nasal, buccal, rectal, transdermal or oral administration, or as a suppository. In addition, the pharmaceutical compositions of the present invention can be made up in a solid form (including without limitation capsules, tablets, pills, granules, powders or suppositories), or in a liquid form (including without limitation solutions, suspensions or emulsions).

The dosage regimen for the compounds of the present invention will vary depending upon known factors, such as the pharmacodynamic characteristics of the particular agent and its mode and route of administration; the species, age, sex, health, medical condition, and weight of the recipient; the nature and extent of the symptoms; the kind of concurrent treatment; the frequency of treatment; the route of administration, the renal and hepatic function of the patient, and the effect desired. In certain embodiments, the compounds of the invention may be administered in a single daily dose, or the total daily dosage may be administered in divided doses of two, three, or four times daily.

The efficacy of a single dose with 100 million total viable cells in an active cell culture of probiotic ARO112 strain is demonstrated in the examples to shorten the length of various pathogenic infections, and increase microbial diversity, provided 48h after last antibiotic treatment. A therapeutically effective dosage level can be ascertained by the skilled artisan by means of animal models of disease, or by reference to the dosages of live bacteria delivered in human clinical trials through similar routes of administration.

In one set of embodiments, the probiotic bacterial composition is formulated for oral delivery into the small or large intestines of the subject, where the majority of the gut microbiota reside. One such embodiment relates to enteric coatings that protect the bacterial composition from high pH in the stomach, and dissolve on reaching the intestines. Examples of such coatings include, without being limited to polymers and copolymers such as eudragit (Evonik).

In a similar embodiment the probiotic composition may be delivered into a specific region of the intestines in the form of buffered sachets, or with a coating that dissolves in a pH range specific to a certain portion of this intestine. For example, a formulation which decomposes in the pH range from 6.8 to 7.5, will favour delivery to the colon (for a full description of targeted delivery to regions of the gastrointestinal tract see Villena et al 2015. Int J. Pharm. 487 (1-2):314-9.)

In yet another similar embodiment, the probiotic composition can be administered specifically to the intestines by means of a time-delay delivery method, which takes into account the time it takes to transit through the stomach, small intestine and colon. Delayed release formulations include hydrogel preparations, and biodegradable, water-soluble, hydrolysable or enzyme degradable polymers. Examples of coating materials that are suitable for delayed-release formulations include, but are not limited to, cellulose-based polymers, acrylic acid polymers, and vinylpolymers.

In another embodiment where the probiotic composition is formulated for delivery to the colon, the formulation includes a coating which can be removed by an enzyme present in the human gut, for example a carbohydrate reductase. Examples of enzyme-sensitive coatings include amylose, xanthan gum and azoploymers.

In embodiments of the invention that relate to rectal administration the probiotic bacterial composition may be formulated for delivery as a suppository, enema or as part of an endo- or colonoscopy procedure.

The therapeutically effective dosage of a the probiotic bacterial composition, pharmaceutical composition, is dependent on the species of the subject, the body weight, age and individual condition, the disorder or disease or the severity thereof being treated. A physician, clinician or veterinarian of ordinary skill can readily determine the effective amount of each of the active ingredients necessary to prevent, treat or inhibit the progress of the disorder or disease.

The probiotics and pharmaceutical compositions of the present invention can contain conventional inert diluents, lubricating agents, or buffering agents, as well as adjuvants, such as preservatives, stabilizers, wetting agents, emulsifiers and buffers, etc. They may be produced by standard processes, for instance by conventional mixing, granulating, dissolving or lyophilizing processes. Many such procedures and methods for preparing pharmaceutical compositions are known in the art, see for example L. Lachman et al. The Theory and Practice of Industrial Pharmacy, 4th Ed, 2013 (ISBN 8123922892).

*Method of Manufacture and Method of Treatment according to the invention*

The invention further encompasses, as an additional aspect, the use of viable *Klebsiella sp.* strain ARO112 as identified herein, for use in a method of manufacture of a medicament for the treatment or prevention of a condition such as dysbiosis, intestinal inflammation, or an inflammatory intestinal disease, or to prevent post-antibiotic pathology.

Similarly, the invention encompasses methods of treatment of a patient having been diagnosed with a disease associated with intestinal inflammation. This method entails administering to the patient an effective amount of a probiotic composition comprising viable *Klebsiella sp.* strain ARO112 as identified herein.

Wherever alternatives for single separable features such as, for example, a coding sequence for the genome of viable *Klebsiella sp.* strain ARO112 used in the composition, or medical indications, or medical symptoms laid out herein as "embodiments", it is to be understood that such alternatives may be combined freely to form discrete embodiments of the invention disclosed herein. Thus, any of the alternative embodiments for a symptom such as inflammation of the intestine may be combined with any of the alternative embodiments of a patient diagnosed with IBD and these combinations may be combined with any medical indication or formulation of the viable *Klebsiella sp.* strain ARO112 composition mentioned herein.

The invention is further illustrated by the following examples and figures, from which further embodiments and advantages can be drawn. These examples are meant to illustrate the invention but not to limit its scope.

## Description of the Figures

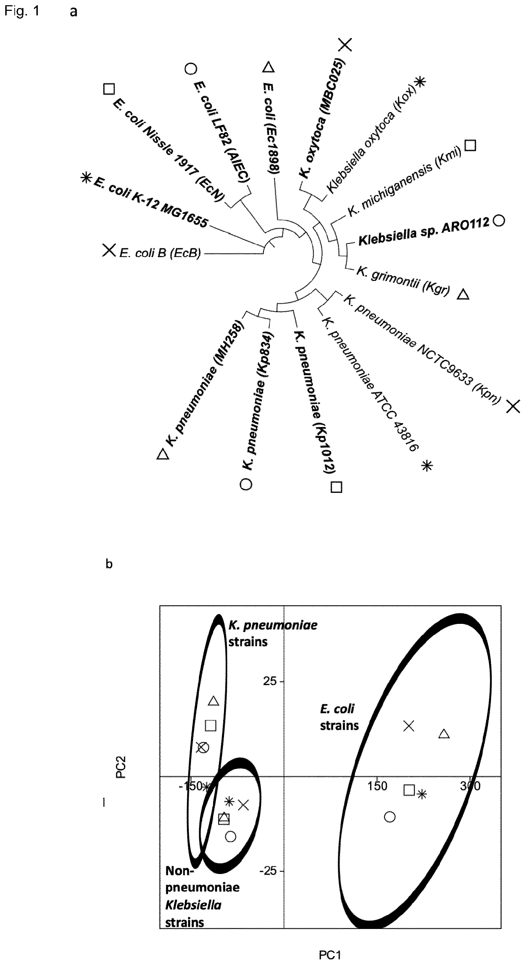

Fig. 1    shows a comparison between Enterobacteriaceae strains from the Klebsiella and Escherichia genera, including clinical isolates of common pathogenic species (Klebsiella pneumoniae and Escherichia coli), a well as commensal species. a) Phylogenetic tree based on whole-genome analysis of 15 indicated strains. b) PCA using the quantification of predicted virulence factors-related genes encoded in their genomes, according to a set of public databases.

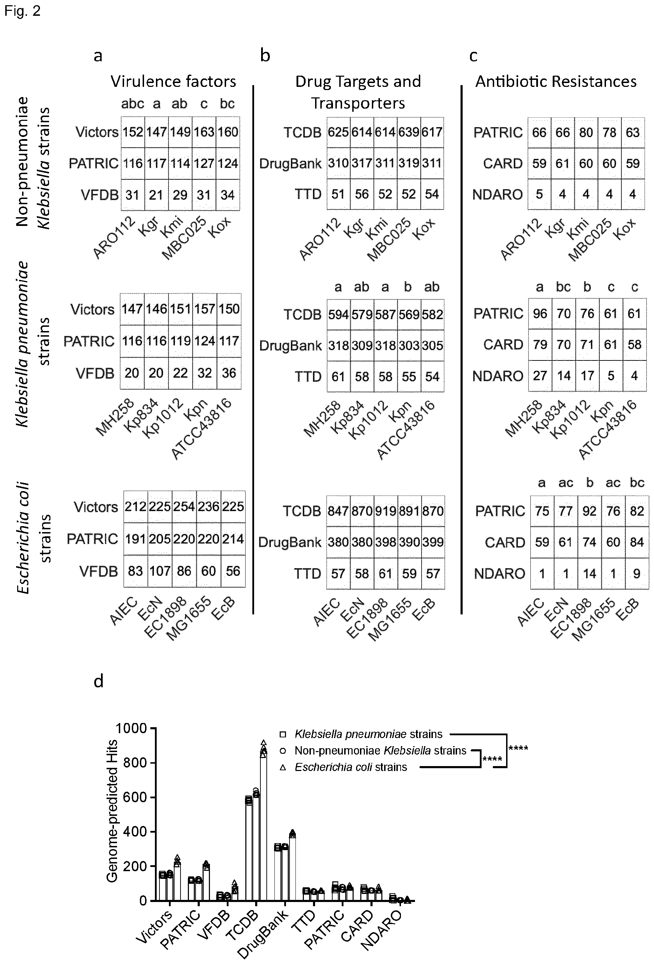

Fig. 2    shows genome-encoded predicted virulence factors using several publicly available databases. Genome analyses were performed in PATRIC web browser, from where the number of hits for the different virulence factors databases were obtained. a) Databases for virulence factors per se (Victors, PATRIC, VFDB), b) databases for drug targets and transporter genes (TCDB, DrugBank, TTD), and c) databases for antibiotic resistances (PATRIC, CARD, NDARO). d) Comparison of number of hits for each database among groups of bacteria. Two-way ANOVA with Tukey's correction for multiple comparisons (**** $p < 0,0001$); different letters denote significant differences ($p < 0.05$) among strains.

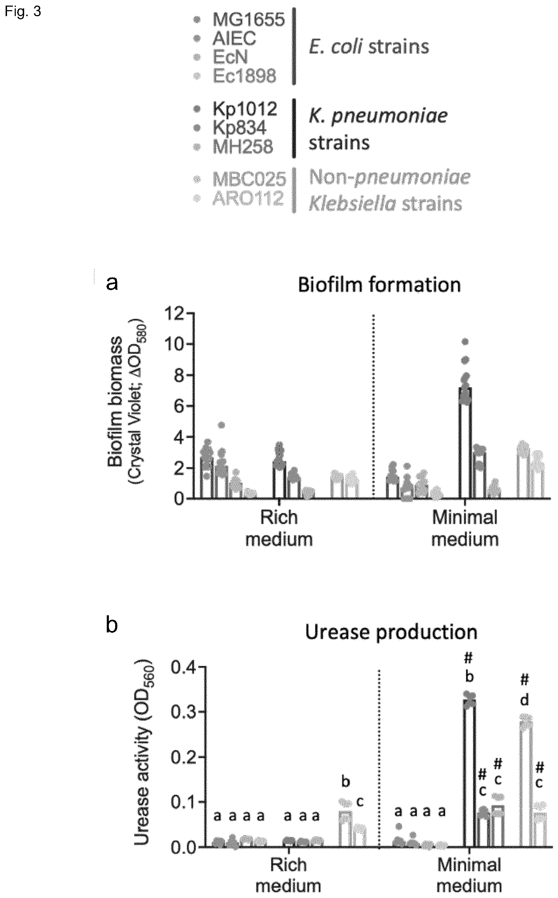

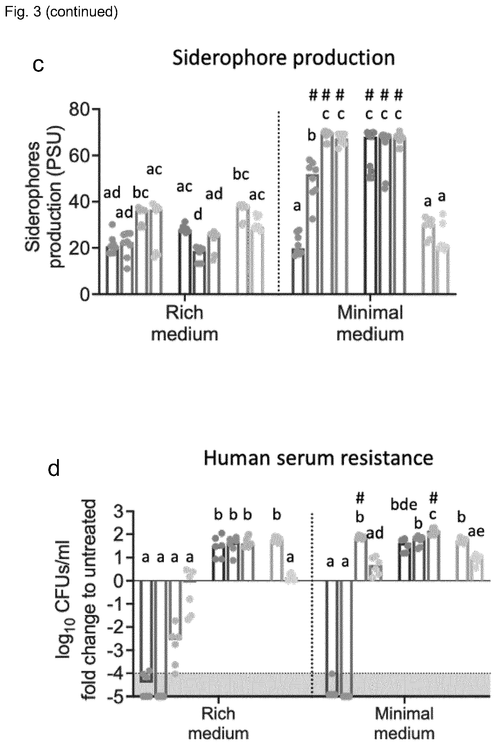

Fig. 3    shows phenotypical evaluation of nosocomial-related virulence factors important for host invasion and colonization. A set of 9 strains (4 E. coli, 3 K. pneumoniae, 2 non-pneumoniae Klebsiella) were tested, both in rich and minimal media, for a) biofilm formation, b) urease activity, c) siderophore production, and d) capacity to resist killing or inhibition by human serum. Different letters denote significant differences among strains within the same medium ($p < 0.05$) and # denotes significant differences between different media for the same strain ($p < 0.05$). Kruskal-Wallis' test with Dunn's correction for multiple comparisons. Biofilms were tested in a total of 15 replicates per group, from 3 independent experiments.

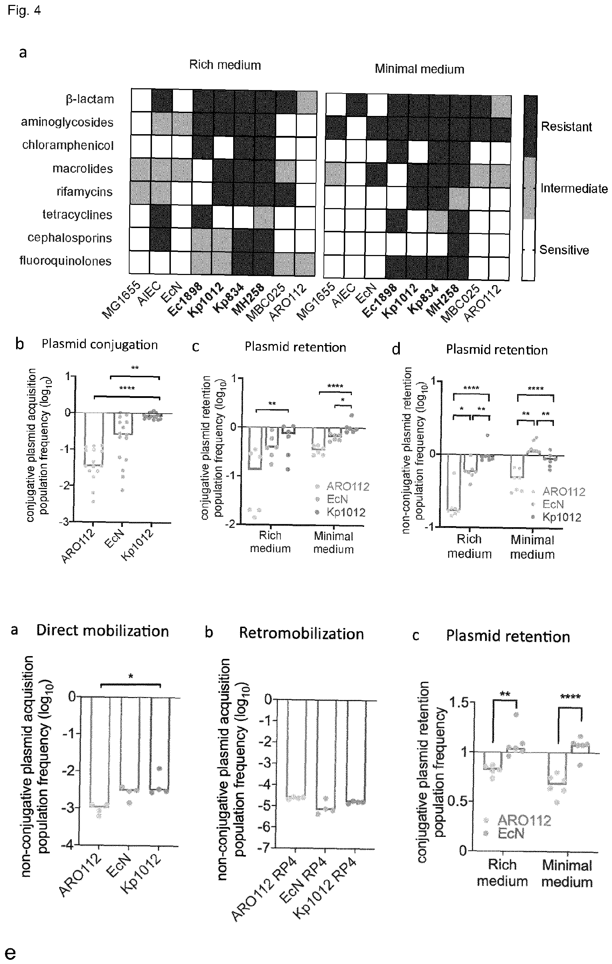

Fig. 4    shows a) antibiotic resistance profiles in rich and minimal media, with Multi-Resistant Enterobacteriaceae strains being represented in bold (resistant to >3 classes of antibiotics). Selected strains were tested for their capacity to b) acquire or c) retain the RP4 conjugative plasmid, in rich and minimal media, as well as the capacity to d) retain a non-conjugative plasmid in rich and minimal media. Kruskal-Wallis' test with Dunn's correction for multiple comparisons b). Two-way ANOVA with Sidak's correction for multiple comparisons (* $p < 0.05$; ** $p < 0.01$; **** $p < 0.0001$; c,d). e) Comparison of HGT capacity for selected strains. Selected strains were tested for their capacity to receive a non-conjugative plasmid (pMP7605) either by direct mobilization or retromobilization with a conjugative plasmid (RP4). ARO112 and EcN were tested for their capacity to retain a natural multi-resistant plasmid acquired by conjugation from the clinical isolate Ec1898. Kruskal-Wallis' test with Dunn's correction for multiple comparisons (* $p < 0.05$). Two-way ANOVA test with Sidak's correction for multiple comparisons (**$p < 0.01$; **** $p < 0.0001$;

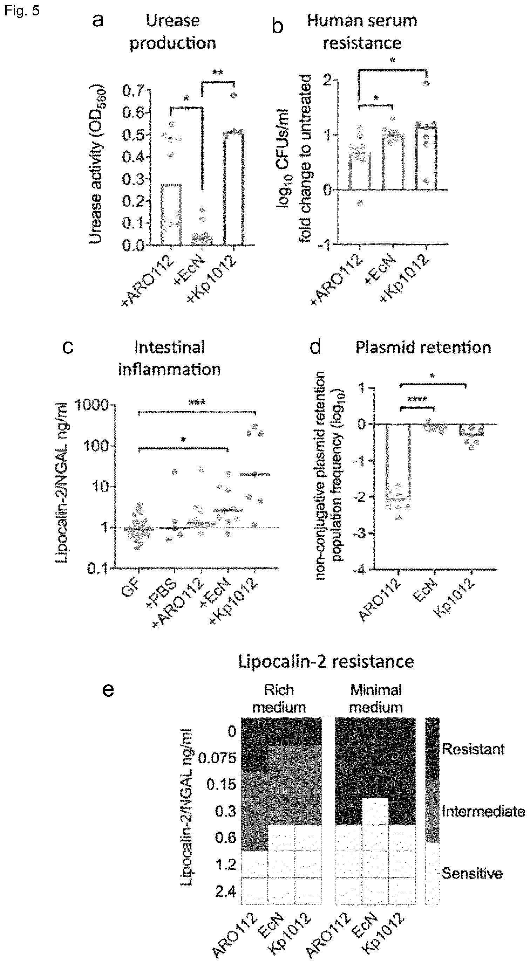

Fig. 5    shows phenotypic testing of bacteria isolated from fecal samples of monocolonized mice. Germ-free mice were monocolonized with each of selected strains for 5 days, after which bacteria were isolated from the fecal samples and tested for a) urease activity and b) resistance to human serum, c) Fecal supernatants were evaluated for Lipocalin-2/NGAL induction, d) Bacteria were also evaluated for plasmid retention upon intestinal colonization. Kruskal-Wallis' test with Dunn's correction for multiple comparisons (* $p < 0.05$; ** $p < 0.01$; *** $p < 0.001$; **** $p < 0.0001$; b-e). Representative of at least 3 independent experiments. Indicated strains were tested for their capacity to grow in the presence of increased concentrations of Lipocalin-2/NGAL in rich or minimal media in 6 replicates, 2 experiments.

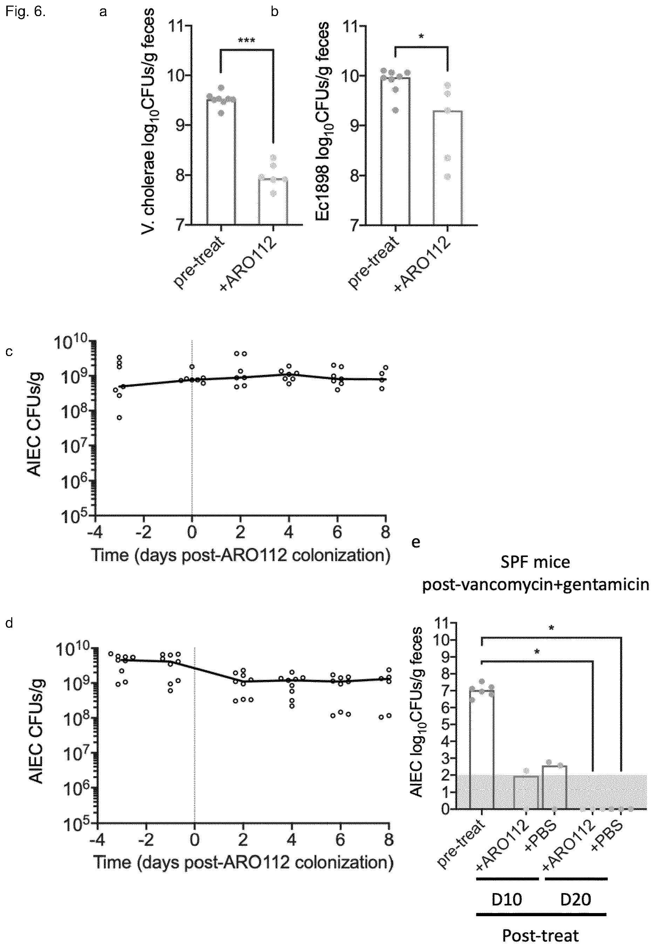

Fig. 6    shows **a)** Loads of *Vibrio cholerae* in gnotobiotic mice before (pre-treat) and after one week of treatment with probiotic (+ARO112). **b)** Loads of multi-resistant *E. coli* strain Ec1898 in gnotobiotic mice before (pre-treat) and after one week of treatment with probiotic strain (+ARO112. **c)** AIEC CFU per gram of feces in gnotobiotic mice before (pre-treat) and after one week of treatment with probiotic strain (+ARO112). **d)** AIEC CFU per gram of feces in SPF wildtype mice treated with streptomycin (5g/L in drinking water) for 2 weeks, before (pre-treat) and after one week of treatment with probiotic strain (+ARO112. **e)** Loads of AIEC strain in SPF wildtype mice previously treated with gentamicin (3 mg/kg/d by gavage) and vancomycin (40 mg/kg/d by gavage) once daily for three consecutive days, before (pre-treat) and after 10 and 20 days of treatment with probiotic strain (+ARO112) or lack thereof (+PBS). Kruskal-Wallis' test with Dunn's correction for multiple comparisons (e) or Mann-Whitney test (a-c) (* p<0.05; ** p<0.01, *** p<0.001). V. cholerae fecal loads in gnotobiotic model were tested in a total of 8 (pre-treat) and 6 (+ARO112) samples from 2 independent experiments (a). Ec1898 fecal loads in gnotobiotic model were tested in a total of 8 (pre-treat) and 5 (+ARO112) samples from 2 independent experiments (b). AIEC fecal loads in gnotobiotic model were tested in a total of 9 (pre-treat) and 9 (+ARO112) samples, from 2 independent experiments (c). AIEC fecal loads in streptomycin-treated model were tested in a total of 14 (pre-treat) and 7 (+ARO112) samples, from 2 independent experiments (d). AIEC fecal loads in gentamicin + vancomycin-treated model were tested in a total of 6 (pre-treat) and 3 (+ARO112) samples, from 1 experiment (e).

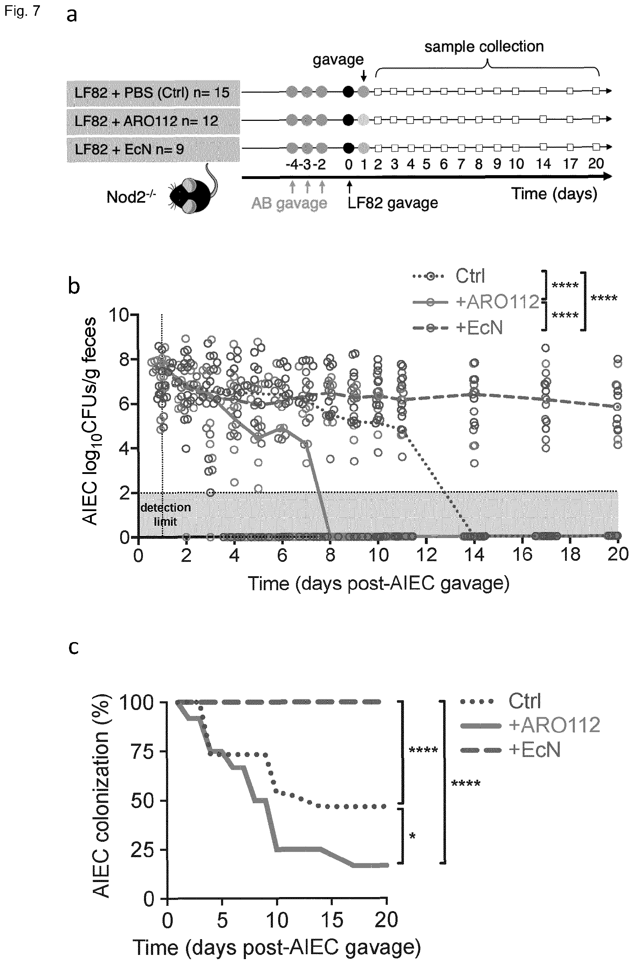

Fig. 7    shows protective capacities of ARO112 and EcN against AIEC colonization in an IBD mouse model previously treated with antibiotics, a) Experimental setup of IBD mice treated with antibiotics, followed by AIEC infection and treated (ARO112, EcN) or not (Ctrl) with a probiotic strain, b) AIEC loads during the experiment, c) Percentage of mice colonized with AIEC throughout the experiment, d) Loads of ARO112 in the IBD mouse model experiment and (below) colonization profile of AIEC (as in c) and ARO112 in IBD mice infected with AIEC and treated with ARO112. Two-way ANOVA with Tukey's correction for multiple comparisons to test overall differences among treatments and with Sidak's correction to test different treatments in each timepoint (* p<0.05, *** p<0.001, **** p<0.0001; 15 (Ctrl), 12 (+ARO112), or 9 (+EcN) animals, from at least 2 independent experiments (a-d).

Fig. 8    shows gut microbiota **a)** richness and **b)** diversity in mice treated or not with probiotics following antibiotic treatment and AIEC infection. **c)** Changes in the relative abundances of most prevalent taxa after antibiotics (post-AB), and after treatment (+ARO112) or lack thereof (Ctrl), compared to level before antibiotics (pre-AB). Changes in relative abundances of **d)** *Pseudomonadota* members, **e)** butyrate-producing bacteria depleted by antibiotic treatment. Kruskal-Wallis' test with Dunn's correction for multiple comparisons **a,b**);Two-way ANOVA test with Tukey's correction for multiple comparisons (* p<0.05; **p<0.01; *** p<0.001; **** p<0.0001; **c-e**). f) PCA of microbiota composition. Changes in relative abundance of g) the indicated taxa associated with butyrate production, h) *Pseudomonodota,* and i) other indicated taxa. Fecal microbiota composition was analyzed from a total of 14 (Nod2$^{-/-}$), 15 (post-AB), 7 (+ARO112), or 7 (Ctrl) animals, from 2 independent experiments (a-d). Two-way Numerical sign (#) denotes significant difference between treatments (ctrl versus ARO112; p<0.1). Plus sign (+) denotes taxa commonly associated with butyrate production. Fecal microbiota composition analyses were performed in a total of 14 (pre-AB), 15 (post-AB), 7 (+ARO112), or 7 (Ctrl) samples, from 2 independent experiments.

Fig. 9    show intestinal inflammation profile of mice upon different probiotic treatments (ARO112, EcN) or lack thereof (Ctrl). a) Intestinal inflammation was measured via lipocalin-2 proxy in fecal supernatants, throughout the experiment. Two-way ANOVA test with Tukey's correction for multiple comparisons (*** p<0.001; **** p<0.0001; a total of 11 (Ctrl), 8 (+ARO112), or 9 (+EcN) samples, from at least 2 experiments.

Fig. 10   shows a) Experimental setup of wildtype mice treated with streptomycin and colonized with AIEC and treated with probiotics ARO112 or EcN. b) Colonization profile of AIEC in streptomycin-treated wildtype mice. c) Inflammation profile of wildtype mice treated with streptomycin and colonized with AIEC pre- (D4) and post-probiotic treatment (D12) with ARO112 or EcN. d) Experimental setup of wildtype ex-germ-free mice colonized with *K. pneumoniae* (Kp1012) and treated with probiotics ARO112 or EcN. e) Colonization profile of *K. pneumoniae* in wildtype ex-germ-free mice. f) Inflammation profile of wildtype ex-germ-free mice colonized with *K. pneumoniae* pre- (D4) and post-probiotic treatment (D12) with ARO112 or EcN. g) Experimental setup of wildtype ex-germ-free mice colonized with *E. coli* (AIEC or Ec1898) and treated with probiotic ARO112. h) Colonization profile of *E. coli* (AIEC or Ec1898) in wildtype ex-germ-free mice. i) Inflammation profile of wildtype ex-germ-free mice colonized with *E. coli* (AIEC or Ec1898) pre- (D4) and post-probiotic treatment (D12) with ARO112. j) Experimental setup of *IL10* KO mice treated with vancomycin and gentamicin and colonized with AIEC and untreated or treated with probiotic ARO112. k) Colonization profile of AIEC in *IL10* KO mice treated with vancomycin and gentamicin. l) Inflammation profile of *IL10* KO mice treated with vancomycin and gentamicin and colonized with AIEC pre- (D0) and post- probiotic treatment (D20) with ARO112, or untreated (Ctrl).

Examples

*Example 1: ARO112 strain presents less aenomicallv-encoded predicted virulence factors than the probiotic EcN.*

A genome-wide comparison of 15 strains: five *E. coli* strains was performed, including the two most common laboratory strains (strain Band K-12 MG1655), one commonly used as probiotic (Nissle 1917), an *E. coli* pathobiont AIEC strain commonly associated with IBD patients (LF82), an *E. coli* strain isolated from a human patient (Ec1898); five *non-pneumoniae Klebsiella* strains including a *K. oxytoca* isolated from murine fecal samples (MBC025), a *K. oxytoca* type strain (DSM5175), a *K. michiganensis* type strain (DSM25444), a *K. grimontii* type strain (06D021) *Klebsiella* ARO112 strain isolated from murine fecal samples; and five *K. pneumoniae* strains, one type strain (NCTC9633), two strains isolated from human samples and commonly used in laboratory research (ATCC43816 and MH258), and two strains isolated from human patients (Kp1012 and Kp834). A phylogenetic tree (Fig. 1a) reveals *E. coli* Nissle 1917 and AIEC strains to be closely related, and further apart from both laboratory strains and the human isolate Ec1898. In the *K. pneumoniae* clade, three human isolates cluster together (MH258, Kp1012, and Kp834), with the other two strains forming another cluster. The *non-pneumoniae Klebsiella* strains show MBC025 clustering with the *K. oxytoca* type strain, and *Klebsiella* ARO112 clustering with *K. grimontii* type strain, the latter cluster being closer to *K. michiganensis* type strain. Upon evaluation of the genome-encoded predicted virulence factors, using 9 databases reported in PATRIC genome browser software as specialty genes in the overview tab for each of the aforementioned genomes (Fig. 2), a Principal Component analysis (PCA) was made (Figure 1b). The resulting genome-predicted virulence factors-based PCA shows a clear separation of the *E. coli* strains from the *Klebsiella* genus, with a slight separation between *K. pneumoniae* and *non-pneumoniae Klebsiella* strains, with ARO112 strain being positioned farther away from the *K. pneumoniae* strains. Interestingly, when analyzing each cluster of strains, according to the databases for the different virulence factors, the databases for virulence factors (Fig. 2) show that only among the *non-pneumoniae Klebsiella* strains there are significant differences, with *K. oxytoca* strains being distinctive from *K. grimontii* type strain, while ARO112 is the only strain that is not significantly different from any of the other five strains in this cluster. *K. pneumoniae* strains show some significant differences among strains when evaluating predicted drug targets and transporters (Fig. 2b). Databases for antibiotic resistances, however, show significant differences among strains both in the *K. pneumoniae* and *E. coli* clusters (Fig. 2c), with 3 *K. pneumoniae* strains and 1 E. coli strain, all isolated from human patients, presenting an overall number of predicted antibiotic resistances.

Using this genome analysis and the results obtained in the databases as a scaffold, a subset of 9 strains were then tested *in laboratorium* for virulence factors usually associated with Enterobacteriaceae, focusing on traits that are can be associated with increased colonization and/or nosocomial infections (Fig. 3a).

*Nosocomial infections and colonization-related virulence factors show context-dependent variability*

Strains including 4 *E. coli* strains (MG1655, AIEC, EcN, Ec1898), 3 *K. pneumoniae* strains (MH254, Kp1012, Kp834), and 2 *non-pneumoniae Klebsiella* strains (MBC025, ARO112), were tested for selected traits that are commonly associated with nosocomial infections and with colonization. Bacterial biofilms are generally considered as virulence factors, an idea promoted by the overwhelming studies performed on pathogenic strains, in contrast to non-pathogenic or commensal strains. The strains were tested for their capacity to form biofilms, both in rich and minimal media. Unsurprisingly, *E. coli* strains are weak biofilm formers, resulting in low biomass communities, regardless of the media tested. The phenotype was measured at 37°C, mimicking the temperature inside the human body, while reports have shown that *E. coli* strains show increased biofilm biomass at lower temperatures, around 25°C, even in rich medium (Mathlouthi, 2018). In fact, in rich medium, most strains resulted in low (EcN, Kp834, MBC025, ARO112) or very low (Ec1898, KpC) biofilm biomass (Fig. 3a), with only MG1655, AIEC, and Kp1012 being moderate biofilm formers (Fig. 3a). Under minimal media, E. coli strains remain as low biofilm formers, similarly to Kp834 (Fig. 3a), while MH254, MBC025, and ARO112 form moderate biofilms and Kp1012 presents a very robust biofilm biomass (Fig. 3a).

Urease production is another phenotype commonly associated with virulence, in particular in Enterobacteriaceae strains. This enzyme catalyzes the hydrolysis of urea into ammonia and carbon dioxide, resulting in formation of phosphate salts and alkalinization of urine. These salts can be encrusted in urinary catheters, allowing bacteria to better adhere and form biofilms, becoming therefore recalcitrant to treatment. Interestingly, when using 24h-grown cultures in rich medium, there was no detectable urease activity, except for MBC025 and, to a lesser extent, ARO112 (Fig. 3b). When the urease activity was measured in cultures grown in minimal medium, however, all *Klebsiella* strains, but not the *E. coli* strains, showed significantly increased values, with Kp1012 presenting the highest activity, followed by MBC025 (a known urease producer species), and low levels by Kp834, MH254, and ARO112 (Fig. 3b). The absence of urease activity in the *E. coli* strains is expected, since this species is a known non-urease producer, even though some clinical isolates of pathogenic *E. coli* (e.g., EHEC) have shown activity. As mentioned before, these two virulence factors can be coupled in urinary infections, for which strains that are proficient in both can be problematic. However, separately, these traits can be purposed towards protective intestinal functions, such as resilience provided by biofilm formation protecting the bacterial community, but also the host epithelial layer from invading bacteria; or the degradation of intestinal urea into carbon dioxide and ammonia, that bacteria can use as nitrogen source, therefore potentially outcompeting invading bacteria.

Another interaction between commensal and pathogenic bacteria and the host is driven by iron availability. Iron is essential for several ubiquitous processes, and the host regulates its availability for example by quenching siderophores, by secreting the protein Lipocalin-2 (Lcn2). Lcn2 is also associated with increased inflammation and is the most commonly used marker for intestinal inflammation assessment in fecal samples. Siderophores that are immune to the quenching capacity of Lcn2 are a further potential virulence factor, and production was measured in rich and minimal media. Rich medium, which is not limited in iron, reduced production of siderophores by all strains (Fig. 3c). An overall increase in siderophore production was present minimal medium. Most strains significantly increased their siderophore production (Fig. 3d), except for MG1566, MBC025, and ARO112, which remained at the same low levels produced when grown in rich medium (Fig. 3c), showing how limiting iron availability does not seem to affect their growth.

Some bacteria can resist innate immune complement killing, for example, via the *O* antigen found in the bacterial lipopolysaccharide. Serum resistance testing in rich and minimal media revealed only the *K. pneumoniae* and MBC025 strains showed resistance to and replication in the presence of pooled human serum (Fig. 3d), indicating their potential capacity to survive and thrive in the bloodstream of patients. On the contrary, most E. coli strains (MG1655, AIEC, EcN) that are very susceptible to human serum, and Ec1898 and ARO112, which are resistant to but do not replicate with human serum (Fig. 3d). Interestingly, testing the same strains grown in minimal medium resulted in a significant increase of resistance and replication for MH258, increasing from 40-fold to 140-fold (Fig. 3d), but more surprising, however, was the change observed for EcN, which presented a strong susceptibility to human serum when grown in rich medium (100-fold decrease), but a strong resistance and replication capacity when grown in minimal medium (70-fold increase; Fig. 3d). Susceptibility to human serum tests are usually performed using rich media to grow bacteria. These results indicate that for some bacteria, like EcN and MH258, resistance is significantly increased when strains are grown in minimal medium instead, a potential caveat on the profiles for this virulence-associated phenotype in previous studies. Finally, ARO112 shows no evidence of killing by the human serum, but it also does not replicate to the levels seen for most of the other strains tested in either medium (Fig. 3d). Because this virulence factor's importance occurs not in the intestinal tract, there is no reason to associate it with a potential protective function of commensal bacteria. Indeed, the ability to replicate in response to human serum is a non-desirable trait even for commensal or protective bacteria, as there is no predicted advantage for the host, while the disadvantage of having replicating bacteria in the bloodstream is evident, either by providing a route for bacteria to infect different organs, or by leading to septicemia.

*Example 3: Resistance to antibiotics in clinical isolated bacteria, unlike non-clinical isolated microbiota members.*

The main clinical problem associated with Enterobacteriaceae are antibiotic resistance, and their efficient ability to acquire antibiotic resistance genes from other bacteria through mechanisms like horizontal gene transfer. Antibiotic profiling of the strains was undertaken in rich and minimal media. MG1655, EcN, MBC025, and ARO112 are not multi-resistant to antibiotics, since they display resistances to less than 3 classes of antibiotics (Fig. 4a), unlike clinically isolated bacteria. Interestingly, both EcN and ARO112 appeared to increase their resistance to the same two classes of antibiotics (aminoglycosides and macrolides) when tested in minimal medium, while sensitive when tested in rich medium (Fig. 4a). The other 5 strains tested showed resistance to 3 (AIEC) or more than 3 (Ec1898, Kp1012, Kp834, MH254) classes of antibiotics, as expected, since these strains are clinical isolates, some of which known for their multi-resistances to antibiotic treatments. Ec1898, Kp1012, and KpC increased the number of classes for which they are resistant when grown in minimal medium, compared to rich medium, with only Kp834 losing a resistant phenotype when grown in minimal medium (Fig. 4a). These results indicate that the stress provided by antibiotics, coupled with the additional stress of minimal medium, can potentiate resistant phenotypes.

The lack of resistances found in the commensal, non-clinical strains, however, does not exclude the potential for antibiotic resistance acquisition, which is common among Enterobacteriaceae when in complex communities, like the gut microbiota. To assess the capacity of ARO112, EcN, and Kp1012 to acquire resistances from other bacteria through horizontal gene transfer, conjugation of a commonly used plasmid (RP4) from a donor strain (*E. coli* K-12 MG1655) was measured. Interestingly, the already multi-resistant strain Kp1012 had the highest acquisition rate, with virtually all CFUs showing acquisition of the plasmid from the donor (Fig. 4b). EcN presented a significantly lower transfer rate, with a median of 25% of the population acquiring the plasmid and consequent antibiotic resistance (Fig. 4b), while ARO112 showed a strong resistance to acquisition, with a median of less that 4% of the population acquiring the plasmid (Fig. 4b). Retention of the acquired plasmids, after five days of daily passages in rich or minimal media, showed virtually no loss of plasmid by the multi-resistant strain Kp1012, and higher loss of the acquired antibiotic resistance by EcN, with 39% and 66% of the population retaining the plasmid in rich and minimal media, respectively (Fig. 4c). Once again, ARO112 shows the highest recalcitrance to antibiotic resistance transfer, with only 13% and 35% of the population maintaining the acquired resistance in rich and minimal media, respectively (Fig. 4c). Although half of known plasmids appear to be non-transmissible they are still able to be shared by bacteria, through conjugation events, either by transferring with a conjugative plasmid from the donor strain (direct mobilization; Fig. 4e) or by using the conjugation route provided by a conjugative plasmid owned by the recipient strain (retromobilization; Fig. 4e). These plasmids can, therefore, find their way into other bacterial cells, providing them with an increased antibiotic resistance profile. The capacity of ARO112, EcN, and Kp1012 to retain a non-conjugative plasmid (pMP7605) carrying an antibiotic resistance showed the conjugative plasmid, Kp1012 was retained the plasmid in virtually all cells, both in rich and minimal media (Fig. 4d). Also similar to the retention of the conjugative plasmid, only 58% of EcN colonies retained the non-conjugative plasmid in rich medium (Fig. 4c), but, unlike the results with the conjugative plasmid, all colonies of EcN retained the non-conjugative plasmid when grown in minimal medium (Fig. 4c). Once again, ARO112 shows the highest plasmid clearance, with 17% and 49% of the population retaining the non-conjugative plasmid when grown in rich or minimal media, respectively (Fig.e 4c).

Direct mobilization and retromobilization of plasmids showed a very low transfer rate by these mechanisms to all three strains, with a significantly lower rate of direct mobilization for ARO112, when compared to Kp1012 (Fig. 4e). The retention capacity of a natural plasmid transferred from one of the multi-resistant clinical isolates, Ec1898, into ARO112 and EcN (Kp1012 was not tested due to its multi-resistant nature) was also measured. Natural plasmids are known to confer some fitness advantages that drive their persistence, despite of cost (Alonso-del Valle, 2021). EcN retained the plasmid in all CFUs tested, while ARO112 was able to lose the plasmid in over 15% (rich medium) and 30% (minimal medium) of the population (Fig. 4e).

*Colonization of the murine gut can alter profiles.*

To determine whether, rich or minimal laboratory media, was replicating more closely the expected phenotypical profiles of bacteria in the mammalian gut, not requiring growth were assayed using bacteria directly isolated from monocolonized mice. Three representative strains- ARO112, EcN, Kp1012 - colonized germ-free mice for 5 days, after which strains were extracted from fecal samples, without selection or growth. Urease activity (Fig. 5a) and resistance to human serum (Fig. 5b) showed while Kp1012 presented the same increased urease activity as minimal medium-grown cultures, and EcN recapitulated the low urease activity of laboratory-grown cultures, ARO112, upon isolation from fecal samples, presents a variable phenotype, with half the samples showing low urease activity, similar to the results obtained with cultures grown in the laboratory, while the other half showed increased urease activity (Fig. 5a). This result, while surprising due to the low levels of urease activity of *in laboratorium*-grown ARO112, indicates a potential competition trait of ARO112, which can be beneficial to both bacteria and host, especially coupled with the low capacity for biofilm formation, unlike Kp1012 that produces high amounts of urease and also forms robust biofilms, presenting an increased urinary tract infection potential.

The same samples were used to test resistance to human serum after gut colonization, with similar results (Fig. 5b) to the ones obtained with laboratory-grown cultures. ARO112 and Kp1012 show similar resistance and replication profiles to the laboratory-grown cultures, gut-isolated EcN is resistant and replicates after 3h of treatment with human serum (Fig. 5b), similarly to the culture grown in minimal medium, and unlike the susceptible culture grown in rich medium. Indeed, ARO112 resistance profile is significantly lower than that of EcN (Fig. 5b), when extracted from the gut of monocolonized mice.

As mentioned above, the host produces Lcn2 as a strategy to control bacterial growth, and increased fecal levels of this protein are a proxy for increased intestinal inflammation. We then tested the ability of each of three selected strains (ARO112, EcN, Kp1012) to induce Lcn2 production and, therefore, gut inflammation, in monocolonized mice. Fecal supernatants after 5 days of monocolonization revealed that colonization with ARO112 did not induce inflammation, unlike colonization with EcN and, to even a greater extent, with Kp1012 (Fig. 5d). Interestingly, the same fecal supernatants were measured for siderophore production, showing low values for all three strains (not shown). The lack of production of considerable levels of siderophores and lack of induction of host Lcn2 and inflammation are host-friendly characteristics for commensal or protective bacteria, since bacterial-induced inflammation is a major health issue, in particular in some gut inflammatory diseases, like IBD and obesity. However low siderophore production and low Lcn2 induction, mean ARO112 might be more susceptible to the increased Lcn2 levels found in the inflammatory gut than strains that do produce higher levels of siderophores and induce Lcn2, such as EcN and Kp1012. ARO112, EcN, or Kp1012, were grown in rich and minimal media, in the absence or upon increasing concentrations of Lcn2. Interestingly, ARO112 showed an overall higher resistance to increased levels of Lcn2 (Fig. 5e), showing that the inability to produce higher amounts of siderophores or to induce inflammation, does not translate to a higher susceptibility to that gut environment. Having the capacity to compete with invading bacteria without the need to compete for iron with other commensal microbes and the host, is an advantage for a potential protective bacterial species.

Non-conjugative plasmid retention was assayed in the murine gut, by colonizing the germ-free mice with each of the strains carrying a non-conjugative plasmid, and quantifying how much of the population retained the plasmid after five days of colonization. Both Kp1012 and EcN maintained the plasmid in almost every cell, while less than 1% of ARO112 cells retained the non-conjugative plasmid (Fig. 5d), showing that the recalcitrance of ARO112 to maintaining acquired antibiotic resistances is exponentiated *in vivo.*

Together, these results indicate that the commonly-used probiotic strain EcN displays higher resistance and replication potential in the presence of human serum, while also presenting considerable levels of antibiotic resistance acquisition and retention.

*Example 4: ARO112 promotes a faster recovery from infection*

Previous work by the inventors has shown that ARO112 is able to partially displace a single bacterial strain, E. *coli* MG1655 from the intestine of ex-germ-free mice (Oliveira, 2020). Surprisingly, in further testing, two additional E. coli strains, the multi-resistant clinical isolate Ec1898, and the IBD-associated pathobiont AIEC, as well as the non-Enterobacteriaceae human pathogen Vibrio cholerae were also displaced faster, with the latter being displaced >37 fold by ARO112 (Fig. 6a). The E. coli strains tested showed different effects, with Ec1898 being displaced >4 fold by ARO112 and >5 fold by EcN (Fig .6b). There was no clinically-relevant displacement by ARO112, nor by EcN, of AIEC under these conditions (Fig. 6c).

This prompted the inventors to test another murine colonization model, the streptomycin-treated SPF wildtype mice, in which ARO112 was also able to drive displacement of MG1655 (Oliveira, 2020). Similar to the results obtained in the germ-free model, neither ARO112 nor EcN were able to displace AIEC (Fig. 6d). The inventors tested a similar murine model of intestinal colonization, SPF wildtype mice, treated with a combination of non-absorbed antibiotics, closely resembling the clinical settings of AIEC colonization, vancomycin and gentamicin, and shown to affect intestinal colonization resistance. Under this regimen of antibiotics, AIEC was displaced from the murine gut regardless of ARO112 presence (Fig. 6e), as expected according to published data (Drouet, 2012). This difference between the antibiotics regimen in the same murine model shows that AIEC permanent colonization is dependent on the microbiota composition resulting from the antibiotic treatment and its subsequent recovery.

Previously published experiments have also shown that a genetic-based murine model for IBD, Nod2$^{-/-}$ mutant mice, under this antibiotic regimen, are more susceptible to AIEC colonization, with a slower displacement profile of this pathobiont (Drouet, 2012). To test the potential of ARO112 or EcN in aiding the slower recovery of colonization resistance microbiota function in this murine model (Fig. 7a), a common IBD-related episode was simulated by Nod2$^{-/-}$ mice treated once daily with antibiotics - vancomycin and gentamicin - through oral gavage on three consecutive days (Drouet, 2012), after which they were gavaged with the AIEC strain LF82. On the following day, mice were either gavaged with PBS (control), ARO112 strain, or the probiotic EcN strain (Fig. 7a). We then followed colonization by AIEC through selective plating, noticing a clearly faster decolonization of AIEC in mice treated with ARO112, with a median drop towards AIEC-free after 8 days of infection (7 days of treatment), compared to mice that received no probiotic treatment, in which a median of 14 days is needed for most mice to resolve the infection (Fig. 7b). Interestingly, treatment with the probiotic EcN resulted in a worse outcome, with all tested mice remaining infected for the 20 days of the experiment (Fig. 7b). When assessing the number of mice that resolve the infection in each condition, it is evident that treatment with EcN promotes a continued infection with AIEC, while the absence of probiotic treatment led to the infection being resolved in half of the population, which is still a worse outcome than the one obtained with the treatment with ARO112, in which 10 out of 12 mice (>83%) became AIEC-free by the end of the experiment (Fig. 7c). Interestingly, ARO112 is itself removed from the intestinal microbiota throughout the experiment, with a median of 10 days post-AIEC infection needed for the loads of ARO112 to drop below detection level in most mice, having disappeared in 9 out of the 12 mice tested (75%) at the end of the experiment (Fig. 7d).

*Example 5: ARO112 treatment promotes microbiota recovery from antibiotic-induced imbalances and recovery of butyrate-producing bacteria*

Antibiotic treatments lead to dysbiotic states of the microbiota, commonly resulting in microbiota communities with reduced diversity and richness, which allow for invasive bacteria, such as AIEC strains, to colonize the intestinal tract. Community parameters showed a recovery of both richness and diversity indices in mice treated with ARO112, unlike the long-lasting decrease of both parameters in the mice that received no probiotic treatment the latter remaining similar to the low levels resulting from the antibiotic treatment (Fig. 8a,b). This microbiota recovery promoted by the treatment with ARO112 (Fig. 8c) can help explain both the faster displacement of AIEC in this group, but also the displacement of ARO112 itself, due to the colonization resistance provided by the recovered microbiota.

Out of the 18 taxa most abundant in our experimental setting, 10 (56%) were depleted by the antibiotics, while 6 (33%) were enriched and 2 (11%) remained unchanged (Fig. 8c). The mice from the control group, absent any probiotic treatment, at the end of the experiment displayed 11 (61%) and 4 (22%) taxa depleted or enriched, respectively, while 3 (17%) taxa showed similar levels to the microbiota before antibiotic treatment (Fig. 8c). When treated with ARO112 strain, mice presented 6 (33%) and 3 (17%) taxa depleted or enriched, respectively, with 9 (50%) taxa recovering levels pre-antibiotics (Fig. 8c). Moreover, of the taxa depleted by antibiotic treatment, 5 were recovered (Fig. 8c). PCA shows that antibiotic treatment (post-AB) resulted in a shift of the microbiota composition farther away from the original microbial community before antibiotic treatment (pre-AB), with mice untreated with probiotics (Ctrl) recover only partially and very stochastically, not clustering, while treatment with ARO112 (+ARO112) results in clustering of the resulting microbiota closer to the sampling before treatment (Fig. 8f).

IBD patients experience blooms of Pseudomonodota, in particular after antibiotic treatment, a reason of concern due to the several members of this phylum that present pathogenic potential. Our experiment shows 3 taxa of Pseudomonodota has being enriched by antibiotics, of which Proteus and Escherichia/Shigella had been significantly reduced at the end of the experiment, regardless of the probiotic treatment or lack thereof (Fig. 8c, d). However, only treatment with ARO112 resulted in low levels similar to before antibiotic treatment. Other Pseudomonodota taxa were not increased by antibiotics, with Klebsiella being enriched upon probiotic treatment, as expected, since this is the taxa of ARO112 (Fig. 8g).

A deeper analysis of the groups of bacteria affected by antibiotic treatment shows that out of the 7 taxa of known butyrate producers, 5 were depleted (Fig. 8e) by the antibiotics, while the other 2 were enriched (Fig. 8g). Treatment with ARO112 resulted in the significant recovery of 3 butyrate producers that had been depleted by antibiotics, Lachnospiraceae, Ruminococcaceae, Oscillibacter (Fig. 8e). Interestingly, mice that were not treated with ARO112 did not recover any of the butyrate producers that were depleted (Fig. 8e). The effect of ARO112 treatment in the recovery and increase of butyrate producers that were depleted by antibiotics is very promising, since studies have tagged butyrate as an important metabolite in IBD, even showing that butyrate orally administered can clinically improve IBD in patients.

ARO112 has the advantage of promoting the producers in the microbiota, therefore potentially leading to a sustained increase of butyrate in the microbiota, instead of the need for administration.

*Example 6: ARO112, unlike EcN, does not induce intestinal inflammation*

Even though the treatment with the potential probiotic ARO112 has shown increased recovery of the microbiota and pathogen clearance, for that treatment to be advantageous for IBD patients, it should not exacerbate IBD symptoms, such as the intestinal inflammation ARO112 reduced production of the Lcn2 proxy for intestinal inflammation in mice absent microbiota, however IBD mice have a resident microbiota and, consequently, a different inflammatory profile. Therefore, the inventors evaluated the intestinal inflammatory response of the IBD mice infected with AIEC and not treated, or treated with ARO112 or EcN. Interestingly, treatment with EcN not only led to persistent infection by AIEC, but also resulted in a lasting increase in intestinal inflammation (Fig. 9a), unlike the control without treatment, for which intestinal inflammation remained at low levels, only significantly and transiently increasing at day 5 of infection (Fig. 9a). More importantly, mice treated with ARO112 showed persistently low levels of intestinal inflammation, never significantly varying from the levels prior to antibiotic treatment (Fig.9a). The inventors tested intestinal inflammation in the several models, noting treating with probiotic ARO112 does not increase inflammation in any of the tested models, independently of the presence or absence of pathogen clearance. In contrast, not treating or treating with probiotic EcN seems to lead to an increase of inflammation (Fig. 10g-i)

*Materials and methods*

*Genome-based prediction of Virulence Factors*

Genomes of all strains were uploaded to the PATRIC BV-BRC browser software (v3.29.20) and analyzed. In each genome's *Overview tab,* the *Specialty Genes* feature was used to quantify the number of predicted genes hits for virulence factors in the plotted databases.

*Biofilm formation*

Biofilm formation in brief, bacterial cultures were grown for 24h in either rich (Lysogenic Broth; LB) or minimal (M9 salts) media at 37°C with shaking. The following day, each culture was washed once in sterile PBS, and final $OD_{600}$ adjusted to 0.05 in the appropriate medium and 200 $\mu$l of culture were dispensed per well in 48-well plates. After adhesion, supernatants were carefully removed and discarded, wells were washed once with 200 $\mu$l of sterile PBS, and 500 $\mu$l of sterile fresh

appropriate medium were carefully dispensed per well. Plates were incubated at 37°C for 24h, after which supernatants were removed and discarded and well bottoms were carefully washed with 500 μl of sterile PBS. Each well was carefully stained with 0.1% Crystal Violet solution and plates were incubated at room temperature for 20 min protected from light. After washing and drying, each well was de-stained with 33% Glacial Acetic Acid and incubated for 15 min at room temperature and read for $OD_{580}$ (peak for Crystal Violet staining), to quantify biofilm biomass.

*Urease activity*

Cultures were grown in either rich or minimal medium for 24h at 37°C with shaking, washed once in sterile PBS and adjusted to an $OD_{600}$ of 1, after which each culture was diluted 20x in urease medium and incubated for 24h at 37°C with shaking. Cultures were then measured at $OD_{560}$ to quantify urease activity. For urease activity measurement of bacteria extracted from fecal samples of monocolonized mice, $2.5 \times 10^7$ cells/ml were used. For urease activity measurement of bacteria extracted from fecal samples of monocolonized mice, cell suspension was prepared as above directly from the extracted bacteria, instead of the grown culture.

*Resistance to Pooled Human Serum*

Cultures were grown in either rich or minimal medium for 24h at 37°C with shaking, washed once in sterile PBS and adjusted to $2 \times 10^7$ cells/ml, after which 25 μl of cell suspension in PBS were added to 75 μl of pooled human serum solution. Cultures were plated for CFUs at 0h and after 3h of static incubation at 37°C, to assess killing, resistance, or proliferation of bacterial cells in the presence of human serum.

*Siderophore production*

Cultures were grown in either rich or minimal medium for 24h at 37°C with shaking, centrifuged and supernatants were recovered and filtered in 0.22 μm filters. Cell-free supernatants were mixed 1:1 (v:v) with Cas-PIPES solution and incubated at room temperature for 20 min, after which $OD_{630}$ was measured to calculate percentage of siderophore production.

$$\% \, siderophores = \frac{(ref - sample) * 100}{ref}$$

*Lipocalin-2/NGAL enzyme-linked immunoassay (ELISA)*

Fecal samples were resuspended in sterile PBS and mechanically homogenized. Samples were centrifuged at 4°C for 15 min at 18,000 xg, and filtered in 0.22

μm filters. ELISA for Lipocalin-2/NGAL (Human Lipocalin-2/NGAL DuoSet ELISA) were performed according to instructions from manufacturer.

*Lipocalin-2 in vitro growth*

Cultures were grown in either rich or minimal medium for 24h at 37°C with shaking, washed once in sterile PBS and adjusted to a final $OD_{600}$ of 0.05 in the appropriate medium with different concentrations of Lipocalin-2 protein. Cultures were grown for 24h in a 96-well plate in a plate reader Biotek Synergy H1 with regular $OD_{600}$ measurements. Final OD600 obtained were compared to the growth in the absence of Lipocalin-2 protein, being considered resistant if growing over 80% of the control, intermediate over 40% of the control and sensitive if below 40% of the control growth.

*Antibiotic profile*

Cultures were grown in either rich or minimal medium for 24h at 37°C with shaking, washed once in sterile PBS and adjusted to a final $OD_{600}$ of 0.05 in the appropriate medium with different concentrations of antibiotics. Cultures were grown for 24h in a 96-well plate in a plate shaker, after which $OD_{600}$ measurements were performed in a plate reader Multiskan. Strains were considered resistant if in the presence of antibiotic could reach at least 80% of the control growth, in the absence of antibiotic. If growth in the presence of antibiotic was more than 40% the control growth, the strain was considered intermediate for resistance, and sensitive if grown less than 40% of control growth.

*Plasmid conjugation experiments*

Cultures were grown in rich medium for 24h at 37°C with shaking, and washed once in sterile PBS. Donor and Recipient strains were thoroughly mixed at a 1:1 ratio (total of $10^8$ cells), centrifuged for 30 sec at 14,000 rpm and resuspended in 20 μl of PBS. Two drops of 10 μl each were placed on top of an agar plate, air-dried, and incubated at 37°C for 1h. After incubation, drops were thoroughly removed with a loop and resuspended in 1 ml of sterile PBS. Serial dilutions were performed and plated to count CFUs for recipient strain with (selective plating with plasmid-borne antibiotic resistance) and without (non-selective or selective plating for recipient's antibiotic resistance plating) tested plasmid. Conjugative plasmid (RP4) harbors resistances to tetracycline (10 μg/ml), kanamycin (50 μg/ml), and ampicillin (100 μg/ml).

*Plasmid retention experiments*

Strains harboring tested plasmids were grown in either rich or minimal medium with antibiotics (for plasmid-borne antibiotic resistance) for 24h at 37°C with shaking, then washed in sterile PBS and adjusted to a final $OD_{600}$ of 0.05 in the appropriate medium without antibiotics. Serial dilutions were plated in selective and non-selective LB agar plates, to assess CFUs with (plasmid retention) and without (plasmid loss) antibiotic resistance. Cultures were incubated at 37°C with shaking for 24h. Every 24h, cultures were diluted 1:100 in fresh appropriate medium and re-incubated, and serial dilutions of the 24h-grown cultures were plated as mentioned before. Experiment ended after 5 days. Non-conjugative plasmid (pMP7506) harbors resistance to gentamicin (30 $\mu$g/ml).

*Fecal microbiota extraction*

Fecal pellets were collected from monocolonized mice for live gut bacteria extraction. Pellets were weighed and 500 $\mu$l of sterile PBS were added before mechanical disruption with a motorized pellet pestle, after which additional 500 $\mu$l of sterile PBS were added. Samples were subjected to four iterations of vortex mixing during 15 sec, centrifugation at 1,000 rpm during 30 sec at room temperature and recovery of 750 $\mu$l of the debris-free, cell-containing supernatants into a new tube, replacing that volume with sterile PBS before the next iteration. After recovery of 3 ml per sample, isolated cells were centrifuged at 4,000 g for 5 min at room temperature, discarding the supernatant and resuspending the cells fractions in 1 ml of sterile PBS supplemented with glycerol and cysteine at final concentrations of 20% and 0.1%.

*Animal experimentation*

All of the mouse experiments performed at Instituto Gulbenkian de Ciência (IGC) were approved by the Institutional Ethics Committee and the Portuguese National Entity (Direção Geral de Alimentação e Veterinária; 015190), which complies with European Directive 86/609/EEC of the European Council. C57BL/6J male mice, either wildtype or Nod2$^{-/-}$, were used at 6-8 weeks of age and were randomly assigned to experimental and control groups. C57BL/6J mice were used for all experiments.

C57BL/6J Nod2$^{-/-}$ mice bred under SPF conditions. Mice were orally gavaged with gentamicin (3 mg/kg/d) and vancomycin (40 mg/kg/d) once daily for three consecutive days (Drouet, 2012). Two days after the last gavage with antibiotics, mice were orally gavaged with ~10^8 CFUs of AIEC strain (LF82-strepR), and the following day were orally gavaged either with PBS (control), or ~10^8 CFUs of ARO112 or EcN strains. Fecal samples were collected at the timepoints indicated in the corresponding graph and plated in selective medium to assess colonization levels of AIEC. Additionally, fecal samples were also used to measure levels of intestinal inflammation (Lipocalin-2/NGAL ELISA) and to assert the intestinal microbiota composition.

C57BL/6J germ-free mice were bred and raised in axenic isolators (La Calhene/ORM) and were later transferred into sterile ISOcages (Tecniplast). Animals were orally gavaged with ~10^8 CFUs of AIEC strain (LF82-strepR), and the following day with ~10^8 CFUs of either ARO112 or EcN strains. Fecal samples were collected at the timepoints indicated in the corresponding graph and plated in selective medium to assess colonization levels of AIEC.

C57BL/6J mice bred under SPF conditions in ventilated cages with high-efficiency particulate air filters. Streptomycin (5 g/l) was maintained ad libitum in the drinking water for 15 days. Streptomycin water was replaced every 3 days. Streptomycin treatment was followed by a period of non-supplemented water during 4 days before AIEC strain (LF82-strepR) was orally gavaged (~10^8 CFUs/mouse). Three days after, either ARO112 strain or EcN strain were orally gavaged (~10^8 CFUs/mouse). Fecal samples were collected at the timepoints indicated in the corresponding graph and plated in selective medium to assess colonization levels of AIEC.

*Fecal microbiota analysis*

Mothur v.1.32.1 was used to process sequences. Sequences were converted to FASTA format. Sequences that were shorter than 220 bp containing homopolymers of longer than 8 bp or undetermined bases with no exact match with the forward and reverse primers, and barcodes that did not complement each other or that did not align with the appropriate 16S rRNA variable region, were not included in the analysis. A quality score above 30 (range, 0 to 40, where 0 represents an ambiguous base) was used to process sequences, which were trimmed using a sliding-window technique over a 50 bp window. Sequences were trimmed from the 3' end until the quality score criterion was met, and were merged after that. Between 20,000 and 50,000 sequences were obtained per sample. 16S rRNA gene sequences were aligned using SILVA template reference alignment. ChimeraSlayer was used to remove potential chimeric sequences. Sequences with distance-based similarity of greater than 97% were joined into the same OTU using the average-neighbour algorithm. All of the samples were rarefied to the same number of sequences (10,000) for diversity analyses. Samples below 10,000 reads were removed from the analysis, which included samples. A Bayesian classifier algorithm with a 60% bootstrap cut-off was used for each sequence; sequences were assigned to the genus level where possible or otherwise to the closest genus-level classification. The top 100 OTUs were used, and taxa plotted are the result of the merge of OTUs with the same classification, being collapsed to 18 different taxa, with lower represented OTUs/taxa being summed and plotted as "other". The Clearcut algorithm was used to infer a phylogenetic tree on the basis of the 16S rRNA sequence alignment, which was used to calculate unweighted and weighted UniFrac distances between each pair of samples.

*Cited prior art documents:*

All scientific publications and patent documents cited in the present specification are incorporated by reference herein.

Santana 2022 - Santana, P.T et al., 2022, Int. J. Mol.Sci.,23,3464.

Small, 2013 - Small, CL., et al., 2013, Nat Commun 4, 1957

(Schultz, 2008) - Schultz, M., 2008, Inflamm Bowel Dis. Jul;14(7):1012-8.

Carmen, 2011 -Carmen, S., 2011, Ulcers, vol. 2011, Article ID 841651, 13 pages

(Suez, 2018); 3) - Suez, J. et al., 2018, Cell 174, 1406-1423

(Montassier, 2021) - Montassier, M. et al., 2021, Nat Microbiol, VOL 6, 1043-1054

Oliveira, 2020 - Oliveira, R.A., et al., 2020, Nat Microbiol, Apr;5(4):630-641.

Moradigaravand 2017 - Moradigaravand D., et al., 2017, mBio 8:e01976-16

Luo 2022 - Luo, X., et al., 2022, Infect Drug Resist, Apr 13;15:1831-1843

Hubbard 2020 - Hubbard, A.T.M, et al., 2020, Microbiologyopen, Jun;9(6):1128-1134.

Zhou 2022 - Zhou, J., et al., 2022, Nat Commun, Jun 14;13(1):3432

Zhilu 2021 - Zhilu, X.,et al., 2021, Gut Microbes, Jan-Dec;13(1)

Huebner 2011 - Huebner, C., et al., 2011, Appl Environ Microbiol., Apr; 77(7): 2541-2544

Drouet, 2012 - Drouet, M., et al., 2012, Inflamm Bowel Dis., Oct;18(10):1923-31

Rolhion 2007 PMID17476674 - Rolhion, N., et al., 2007, Inflammatory Bowel Diseases, Volume 13, Issue 10, 1, Pages 1277-1283

Palmela 2019 PMID 29141957 - Palmela C, et al. Gut 2017;0:1-14.

(Lev-Tzion 2017). PMID 26056382 - Lev-Tzion, R., et al., 2017, Digestion, 95:310-313

(Xiong 2015). PMID 28564649 - Xiong H, et al., 2015, Infect Immun 83:3418 -3427

## Claims

1. A probiotic composition comprising as the only active bacterial ingredient viable *Klebsiella sp.* strain ARO112 bacteria.

2. A probiotic composition comprising as the only active bacterial ingredient viable *Klebsiella sp.* strain ARO112 bacteria, for use as a medicament.

3. The probiotic composition for use according to claim 2, for use in promoting bacterial diversity in a mammalian subject, particularly in a human subject.

4. The probiotic composition for use according to claim 2 or 3, for use in preventing, or treating intestinal dysbiosis in a mammalian subject, particularly a human subject.

5. The probiotic composition for use according to any of the claims 2 to 4, for use in preventing, or treating intestinal inflammation in a mammalian subject, particularly a human subject.

6. The probiotic composition for use according to any one of the claims 2 to 5, for use in preventing, or treating an intestinal infection by a pathogenic bacterial species in a mammalian subject, particularly a human subject.

7. The probiotic composition for use according to any one of the claims 2 to 6, wherein the pathogenic bacterial species is an antibiotic-resistant bacterial species, particularly a multi-drug resistant bacterial species.

8. The probiotic composition for use according claims 6 or 7, wherein the pathogenic bacterial species is:

   - a member of a genus selected from *Escherichia, Citrobacter,* or *Salmonella.*
   - *Clostridioides difficile,*
   - a vancomycin-resistant *Enterococcus* species; and/or
   - a multi-drug resistant species of pathogenic bacteria selected from *Enterococcus faecium, Staphylococcus aureus, Klebsiella pneumoniae, Acinetobacter baumannii, Pseudomonas aeruginosa, Enterobacter species,* and *E. coli.*

9. The probiotic composition for use according to any one of the claims 6 to 8, wherein the pathogenic bacterial species is adherent-invasive *E. coli* (AIEC), enterohemorrhagic *Escherichia coli* (EHEC), enteroaggregative *E. coli (EAEC),* enteropathogenic *E. coli (EPEC)* or uropathogenic *E. coli* (*UPEC),* particularly wherein the pathogenic bacteria is the AIEC *E. coli* strain LF82.

10. The probiotic composition for use according to any one of the claims 2 to 9, for use in a human subject who has received a therapeutic course of antibiotics in the previous month, particularly in the previous 48 hours.

11. The probiotic composition for use according to claim 10, wherein the therapeutic course of antibiotics comprises vancomycin, gentamicin, metronidazole, ciprofloxacin, and/or rifaximin.

12. The probiotic composition for use according to any of the claims 2 to 11, for use in a patient diagnosed with an intestinal inflammatory disease.

13. The probiotic composition for use according to claim 12, wherein the intestinal inflammatory disease is an inflammatory bowel disease, particularly Crohn's disease, or ulcerative colitis.

14. The probiotic composition, or the probiotic composition for use according to any one of the claims 1 to 13, wherein the *Klebsiella sp.* strain ARO112 bacteria are **characterized by** a genome >97% >98%, >99%, >99.5%, or particularly >99.9% similar to the genome of the *Klebsiella sp.* ARO112 strain deposited in the Sequence Read Archive (SRA) of the NCBI database under the BioProject ID. PRJNA590204.

15. The probiotic composition for use according to any of the claims 2 to 14, wherein the probiotic composition is administered orally.

Fig. 1    a

b

Fig. 2

Fig. 3

- MG1655
- AIEC        E. coli strains
- EcN
- Ec1898

- Kp1012      K. pneumoniae
- Kp834       strains
- MH258

- MBC025      Non-pneumoniae
- ARO112      Klebsiella strains

a    **Biofilm formation**

b    **Urease production**

Fig. 3 (continued)

c  **Siderophore production**

d  **Human serum resistance**

Fig. 4

Fig. 5

Fig. 6.

Fig. 7 a

Fig. 7 (continued)

d

d

Fig. 8

a

b

Fig. 8 (continued)

**c**

Fig. 8 (continued)

**d**

**e**

Fig. 8 (continued)

f

g

Fig. 8 (continued)

h

i

Fig. 9

a

Fig. 10

Fig. 10 (continued)

g.

AIEC
Ec1898         ARO112

AIEC (+ARO112) n= 9

Ec1898 (+ARO112) n= 8

0      4              12

WT
GF

Time (days)

j.

AIEC    ARO112
PBS (Ctrl)

AIEC (+ARO112) n= 3

AIEC (+EcN) n= 3

0   1              20

IL10 KO SPF
vancomycin+
gentamicin-
treated

Time (days)

h.

$E.\ coli\ \log_{10}$ CFUs/g feces

Time (days post-colonization)

AIEC (+ARO112)
Ec1898 (+ARO112)

k.

AIEC $\log_{10}$ CFUs/g feces

AIEC (+ARO112)
AIEC (Ctrl)

Time (days post-AIEC colonization)

i.

Lipocalin-2/NGAL change from D0 ng/g feces

pre-  post-  pre-  post-

Probiotic treatment

l.

Lipocalin-2/NGAL change from D0 ng/g feces

*

pre-  post-  pre-  post-

Probiotic treatment

34

Europäisches
Patentamt

European
Patent Office

Office européen
des brevets

**EUROPEAN SEARCH REPORT**

Application Number

EP 23 18 4673

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X,D | OLIVEIRA RITA A. ET AL: "Klebsiella michiganensis transmission enhances resistance to Enterobacteriaceae gut invasion by nutrition competition", NATURE MICROBIOLOGY, vol. 5, no. 4, 1 April 2020 (2020-04-01), pages 630-641, XP055859342, DOI: 10.1038/s41564-019-0658-4 Retrieved from the Internet: URL:https://www.nature.com/articles/s41564-019-0658-4.pdf> | 1-4,6-15 | INV. A61K35/00 A61P1/00 |
| A | * figure 2 * | 5 | |
| A | GEORGINA L HOLD: "Role of the gut microbiota in inflammatory bowel disease pathogenesis: What have we learnt in the past 10 years?", WORLD JOURNAL OF GASTROENTEROLOGY, vol. 20, no. 5, 1 January 2014 (2014-01-01), page 1192, XP055252895, CN ISSN: 1007-9327, DOI: 10.3748/wjg.v20.i5.1192 | 1-12 | |
| | | | TECHNICAL FIELDS SEARCHED (IPC) |
| | | | A61K A61P |
| A,D | SCHULTZ MICHAEL: "Clinical use of E. coli Nissle 1917 in inflammatory bowel disease :", HHS PUBLIC ACCESS AUTHOR MANUSCRIPT, vol. 14, no. 7, 1 July 2008 (2008-07-01), pages 1012-1018, XP093108400, US ISSN: 1078-0998, DOI: 10.1002/ibd.20377 | 1-12 | |

The present search report has been drawn up for all claims

| Place of search | Date of completion of the search | Examiner |
|---|---|---|
| Munich | 5 December 2023 | Vandenbogaerde, Ann |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

& : member of the same patent family, corresponding
document

EPO FORM 1503 03.82 (P04C01)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description

- **SAMBROOK et al.** Molecular Cloning: A Laboratory Manual. Cold Spring Harbour Laboratory Press, 2012
- **AUSUBEL et al.** Short Protocols in Molecular Biology. John Wiley & Sons, Inc., 2002
- Remington: the Science and Practice of Pharmacy
- **SMITH** ; **WATERMAN**. *Adv. Appl. Math.*, 1981, vol. 2, 482
- **NEEDLEMAN** ; **WUNSCH**. *J. Mol. Biol.*, 1970, vol. 48, 443
- **PEARSON** ; **LIPMAN**. *Proc. Nat. Acad. Sci.*, 1988, vol. 85, 2444
- **ALTSCHUL et al.** *J. Mol. Biol.*, 1990, vol. 215, 403-410
- **VILLENA et al.** *Int J. Pharm.*, 2015, vol. 487 (1-2), 314-9
- **L. LACHMAN et al.** The Theory and Practice of Industrial Pharmacy. 2013
- **SANTANA, P.T et al.** *Int. J. Mol.Sci.*, 2022, vol. 23, 3464
- **SMALL, CL et al.** *Nat Commun*, 2013, vol. 4, 1957
- **SCHULTZ, M.** *Inflamm Bowel Dis.*, July 2007, vol. 14 (7), 1012-8
- **CARMEN, S.** *Ulcers*, 2011, vol. 2011, 13
- **SUEZ, J. et al.** *Cell*, 2018, vol. 174, 1406-1423
- **MONTASSIER, M. et al.** *Nat Microbiol*, 2021, vol. 6, 1043-1054
- **OLIVEIRA, R.A. et al.** *Nat Microbiol*, April 2020, vol. 5 (4), 630-641
- **MORADIGARAVAND D. et al.** *mBio*, 2017, vol. 8, e01976-16
- **LUO, X. et al.** *Infect Drug Resist*, 13 April 2022, vol. 15, 1831-1843
- **HUBBARD, A.T.M et al.** *Microbiologyopen*, June 2020, vol. 9 (6), 1128-1134
- **ZHOU, J. et al.** *Nat Commun*, 14 June 2022, vol. 13 (1), 3432
- **ZHILU, X.** *Gut Microbes*, January 2021, vol. 13 (1)
- **HUEBNER, C. et al.** *Appl Environ Microbiol.*, April 2011, vol. 77 (7), 2541-2544
- **DROUET, M. et al.** *Inflamm Bowel Dis.*, October 2012, vol. 18 (10), 1923-31
- **ROLHION, N. et al.** *Inflammatory Bowel Diseases*, 2007, vol. 13 (10), 1277-1283
- **PALMELA C et al.** *Gut*, 2017, vol. 0, 1-14
- **LEV-TZION, R. et al.** *Digestion*, 2017, vol. 95, 310-313
- **XIONG H et al.** *Infect Immun*, 2015, vol. 83, 3418-3427